# EUROPÄISCHE PATENTSCHRIFT

(11) **EP 0 665 836 B1**
(45) Veröffentlichungstag und Bekanntmachung des Hinweises auf die Patenterteilung: **29.04.1998**
(21) Anmeldenummer: 93921924.2
(22) Anmeldetag: 06.10.1993
(51) Int. Cl.: C07D 239/30, C07D 213/61, C07D 241/16, C07C 43/225

(54) **BIFUNKTIONELLE VORPRODUKTE ZUR HERSTELLUNG VON FLUSSIGKRISTALLEN**
BIFUNCTIONAL INTERMEDIATES FOR USE IN THE MANUFACTURE OF LIQUID CRYSTALS
INTERMEDIAIRES BIFONCTIONNELS POUR FABRIQUER DES CRISTAUX LIQUIDES

(30) Priorität: 26.10.1992 DE 4236104
(43) Veröffentlichungstag der Anmeldung: 09.08.1995
(73) Patentinhaber: HOECHST AKTIENGESELLSCHAFT, 65926 Frankfurt am Main (DE)
(72) Erfinder: SCHLOSSER, Hubert, D-61479 Glashütten/Taunus (DE); WINGEN, Rainer, D-65795 Hattersheim am Main (DE); MANERO, Javier, D-65931 Frankfurt am Main 80 (DE)
(86) Internationale Anmeldenummer: EP9302732
(87) Internationale Veröffentlichungsnummer: WO9410152

(56) Entgegenhaltungen:
- EP-A- 0 225 195
- EP-A- 0 284 093
- EP-A- 0 313 284
- EP-A- 0 313 338
- EP-A- 0 339 252
- EP-A- 0 354 434
- EP-A- 0 360 042
- EP-A- 0 360 622
- EP-A- 0 394 906
- EP-A- 0 439 089
- EP-A- 0 517 498
- WO-A-89/06678
- WO-A-91/05029
- GB-A- 2 197 868
- US-A- 4 835 274

## Beschreibung

Die ungewöhnliche Kombination von anisotropem und fluidem Verhalten der Flüssigkristalle hat zu ihrer Verwendung in elektrooptischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffe ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristallmischungen und somit auch an einer Vielzahl mesogener Verbindungen unterschiedlicher Struktur. Dies gilt sowohl für die Anwendungen, bei denen nematische Flüssigkristallphasen verwendet werden, als auch für solche mit smektischen Flüssigkristallphasen.

Auch die Herstellungsverfahren der Komponenten derartiger FlüssigkristallMischungen haben sich ständig steigenden Anforderungen zu stellen, vor allem hinsichtlich der Auswirkungen auf die Ökologie, aber auch im Hinblick auf Prozeßökonomie. Fast immer bestehen die Flüssigkrisiallmischungen aus mindestens zwei verschiedenen Substanzklassen, und sehr häufig findet man in einer bestimmten Substanzklasse mindestens zwei Homologe, die sich z.B. in der Kettenlänge eines Alkyl- oder Alkyloxy-Substituenten unterscheiden (s. z.B. EP-A 497 176, US- 5,026,506, EP-A 495 686, EP-A 319 167, EP-B 317 587, EP-A 316 181, EP-A-315 958).

Es ist deshalb schon nach Wegen gesucht worden, die Synthesen verschiedener, aber über gemeinsame Teilstrukturen verfügende Substanzklassen auf die Basis gemeinsamer Vorprodukte zu stellen.

So werden in der EP-A 354 434 Derivate der Borsäure, darunter auch Boronsäuren, beschrieben, die mit bestimmten Halogenverbindungen unter Katalyse durch Metallverbindungen zu Flüssigkristallverbindungen umgesetzt werden.

Ein wesentlicher, die kostengünstige Produktion beeinträchtigender und durch zusätzliche Prozeßstufen ökologisch zu bedenkender Nachteil der in EP-A 354 434 beschriebenen Verbindungen ist jedoch die Tatsache, daß bei der weiteren Umsetzung zu flüssigkristallinen Verbindungen zur Herstellung von mehr als einem Homologen einer Substanzklasse jeweils mehr als ein Borsäurederivat bzw. eine Halogenverbindung erforderlich ist, da das Substitutionsmuster der gewünschten Zielmoleküle schon in den Ausgangsverbindungen für die Borsäurederivate bzw. Halogenverbindungen festgelegt wird und in den folgenden Reaktionsschritten eine Homologisierung nicht mehr möglich ist.

Für die Herstellung unterschiedlicher Substanzklassen - worunter hier beispielsweise nicht nur Phenylpyridin versus Phenylpyrimidin, sondern beispielsweise auch 5-Alkyl- versus 5-Alkoxy- versus 5-Alkyloxycarbonylversus 5-Alkylcarbonyloxy - pyrimidin verstanden werden, da deutliche Unterschiede hinsichtlich mesogener Eigenschaften, Stabilität, Synthese und damit nicht zuletzt auch der Vorprodukte bestehen - müssen nach dem Stand der Technik jeweils unterschiedliche Vorprodukte hergestellt werden.

In der prioritätsälteren, nicht vorveröffentlichten EP-A 0 517 489, die gemäß Art. 54(3)(4) Stand der Technik für die Vertragsstaaten DE, FR, GB darstellt, ist die Verbindung 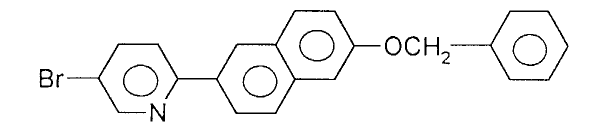 als Zwischenprodukt zur Synthese von Flüssigkristallverbindungen beschrieben.

Die beschriebenen Nachteile werden behoben durch die erfindungsgemäßen bifunktionellen Verbindungen der Formel (I), 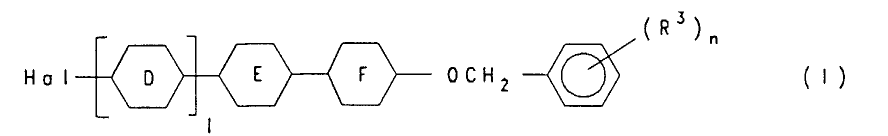 worin bedeuten: 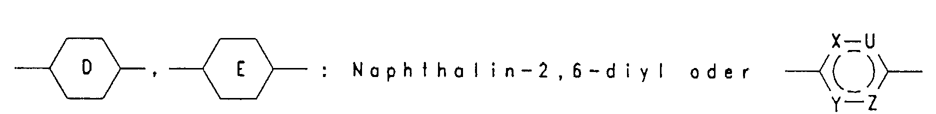 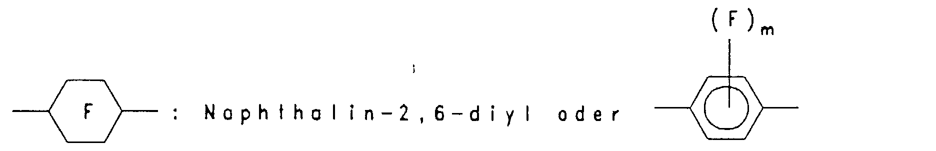 U, X, Y, Z : -CH =, -CF = und -N =, mit der Maßgabe, daß -CF= bzw. -N = jeweils nur höchstens zweimal pro Sechsring vertreten sein dürfen und daß in einem Sechsring -CF = und -N = nicht zugleich zweimal vertreten sein dürfen;
- Hal:: Cl, Br oder I; oder H, wenn X oder/und Y -CF = sind
- R³:: CH₃ oder OCH₃
- I:: 0 oder 1
- m, n:: 0, 1, 2 oder 3,
wobei für die Vertragsstaaten DE, FR, GB die folgende Verbindung ausgenommen ist: 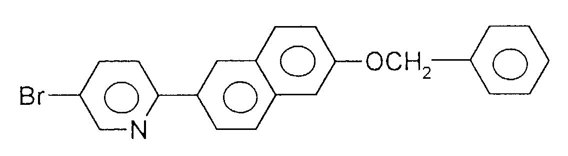 Bevorzugt werden die Verbindungen, in denen
- I =: 0
- U,X,Y,Z:: mindestens einmal -N = und höchstens einmal -CF =, ansonsten -CH =
- m:: 0, 1 oder 2
- n:: 0, 1 oder 2
- R³:: CH₃ oder OCH₃
- Hal:: Br oder I
bedeuten.

Besonders bevorzugt werden die Verbindungen, in denen
- I =: 0
- U,X,Y,Z:: ein - oder zweimal -N =, ansonsten -CH =
- m:: 0, 1, 2 oder 3
- n:: 0
- Hal:: Br oder I
bedeuten.

Ganz besonders bevorzugt werden die Verbindungen, in denen
- I =: 0
- U,X,Y,Z:: ein - oder zweimal -N =, ansonsten -CH =
- m,n:: 0
- Hal:: Br
bedeutet.

Insbesondere bevorzugt sind die Verbindungen 1a -c.

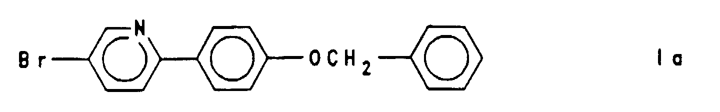

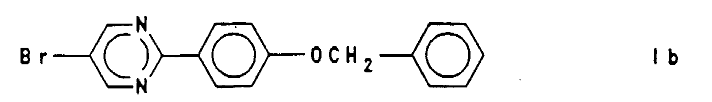

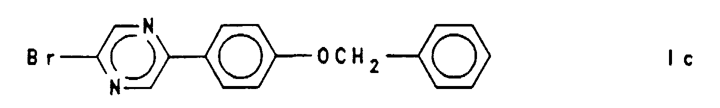

Die erfindungsgemäßen Verbindungen können über ihre zwei unterschiedlichen Funktionalitäten - die Halogen- und die Benzylether-Funktion - sukzessiv selektiv derivatisiert werden.

So kann über die Halogenfunktion nach metall-katalysierten Verfahren, wie sie z.B. in DE-C 3 930 663 und EP-A 354 434 beschrieben sind, mit Boronsäuren der Formel II, 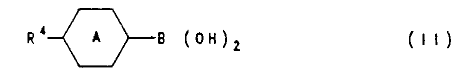 worin R⁴ = H, Alkyl mit 1 bis 18 C-Atomen, worin auch eine oder mehrere nicht benachbarte -CH₂-Gruppen durch -O-, -C(O)-, -CH=CH-, -OC(O)- und -Si(CH₃)₂- ersetzt sein können, und 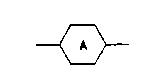 1,4-Phenylen, gegebenenfalls 1, 2 oder 3 mal durch F substituiert, bedeuten,
zu Intermediaten der Formel (III) 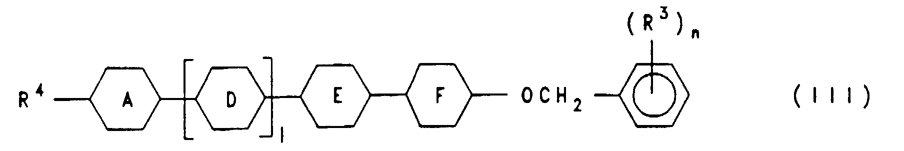 umgesetzt werden,
worin die Symbole die in (I) und (II) genannten Bedeutungen besitzen.

Bevorzugt ist die Umsetzung, beispielsweise zu Verbindungen der formel (III), nach einem in der deutschen Patentanmeldung P 42 36 103.6 mit dem Titel "Verfahren zur Kreuzkupplung von aromatischen Boronsäuren mit aromatischen Halogenverbindungen oder Perfluoalkylsulfonaten" vorgeschlagenen Verfahren, wobei die erfindungsgemäße Verbindung mit aromatischen Halogenverbindungen oder Perfluoralkylsulfonaten in Gegenwart einer Base und katalytischer Mengen metallischem, ggf. auf ein Trägermaterial aufgetragenem Palladium gekoppelt werden, dadurch gekennzeichnet, daß dem Reaktionsgemisch eine Base und katalytische Mengen eines Liganden zugegeben werden.

Ferner ist es möglich, die erfindungsgemäßen Verbindungen der Formel I nach metall-katalysiertem Verfahren, wie sie z.B. in DE-C 3 930 663 und EP-A 354 434 beschrieben sind, mit alkylmetallorganischen Verbindungen zu Intermediaten der Formel (IV) umzusetzen, 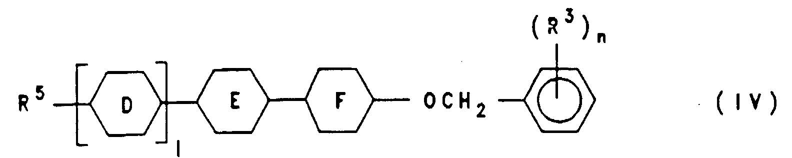 worin
- R⁵: Alkyl mit 1 bis 18 C-Atomen, worin auch eine oder mehrere nicht benachbarte -CH₂-Gruppen durch -O-, -CH = CH- oder -Si(CH₃)₂- ersetzt sein können, wobei -O- nicht direkt am Kern angebunden sein darf,
bedeutet und die übrigen Symbole die in der Formel (I) angegebenen Bedeutungen haben.

Zusätzlich ist es möglich, durch Umsetzung mit OH-Nucleophilen die Halogenfunktion in den Verbindungen der Formel (I) in eine OH-Gruppe zu überführen.

Erfindungsgemäße Verbindungen der Formel V, 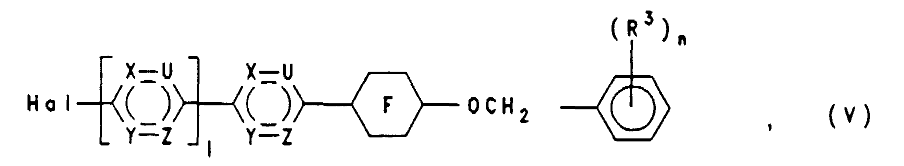 worin
die Symbole die in Formel (I) angegebenen Bedeutungen haben, mit der Maßgabe, daß in 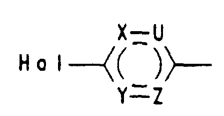 mindestens U und/oder Z -N = sind und
X und/oder Y nicht -N = sein dürfen,
werden vorzugsweise nach einem in der deutschen Patentanmeldung P 42 36 102.8 mit dem Titel "Verfahren zur Herstellung von Hydroxyheteroaromaten" vorgeschlagenen Verfahren in einem Lösungsmittel bei Atmosphärendruck mit einem Metallhydroxid unter Verwendung katalytischer Mengen Schwefels zu Verbindungen der Formel VI, 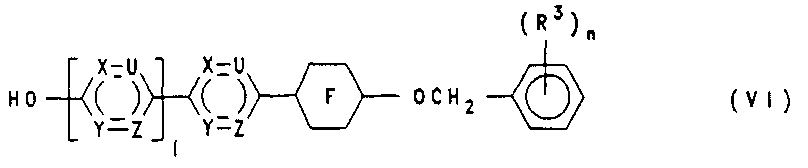 worin die Symbole die in Formel (V) angegebenen Bedeutungen haben, umgesetzt.

Diese Intermediate (VI) können nach Standardmethoden der Synthese von Alkylarylethern oder Alkansäurearylestern in Intermediate der Formel (VII) überführt werden, worin 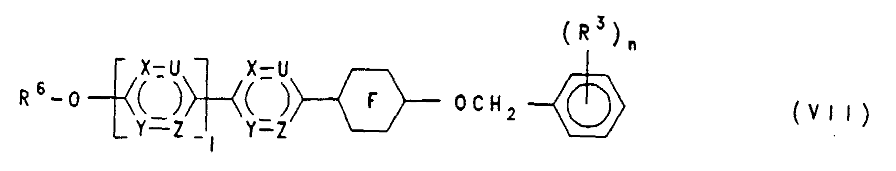
- R⁶: Alkyl mit 1 bis 18 C-Atomen, worin auch eine oder mehrere nicht benachbarte -CH₂-Gruppen durch -O-, -C(=O)-, -CH=CH- oder -Si(CH₃)₂- ersetzt sein können,
bedeutet und die übrigen Symbole die in Formel (V) angegebenen Bedeutungen haben.

Ferner können Intermediate der Formel (VI) nach Standardmethoden durch Umsetzung mit Carbonsäuren bzw. Carbonsäurederivaten (z.B. Halogenide oder Anhydride) in Intermediate der Formel (VIII) überführt werden, worin bedeuten 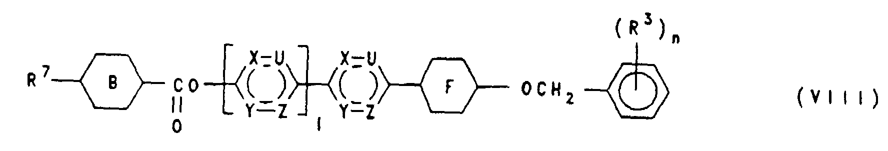
- R⁷: Alkyl mit 1 bis 18 C-Atomen, worin auch ein oder mehrere nicht benachbarte -CH₂-Gruppen durch -O-, -CH=CH- oder -Si(CH₃)₂- ersetzt sein können, 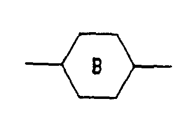
1,4-Phenylen, gegebenenfalls 1, 2 oder 3 mal durch F substituiert, oder 1,4-Cyclohexylen oder 1,3-Dioxan-2,5-diyl.

Die Intermediate (III), (IV), (VI), (VII) und (VIII) sind ebenfalls Gegenstand der vorliegenden Erfindung.

Durch Spaltung der Benzylether-Funktion in den Intermediaten (I), (III), (IV), (VII) und (VIII) nach Standardmethoden (z.B. beschrieben in: T.W.Greene, P.G.M.Wuts: Protective Groups in Organic Synthesis, J.Wiley & Sons, New York, 1991, pp.156-160) werden neue Intermediate mit phenolischer OH-Funktion, die ebenfalls Gegenstand der vorliegenden Erfindung sind, erzeugt:

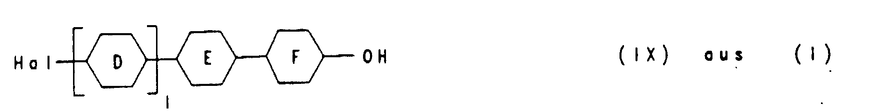

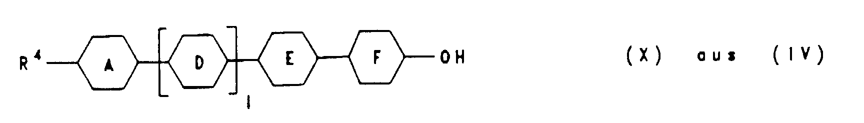

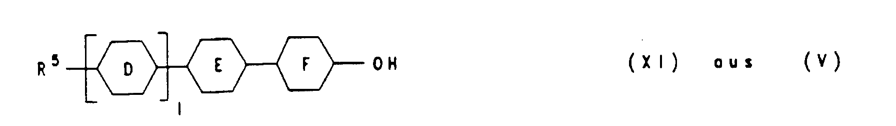

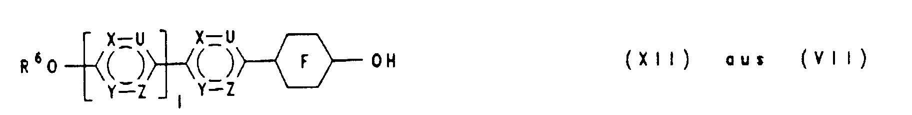

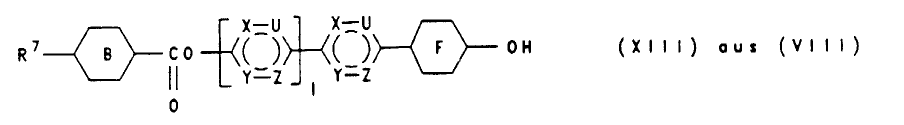

Diese phenolischen Verbindungen der Formeln (IX) bis (XIII) können nach Standardmethoden in zahlreiche Typen von Komponenten für Flüssigkristallmischungen überführt werden. Zum Beispiel können durch Umsetzung mit Alkylhalogeniden bzw. äquivalenten Alkylierungsmitteln Arylalkylether der Formel (XIV) erhalten werden 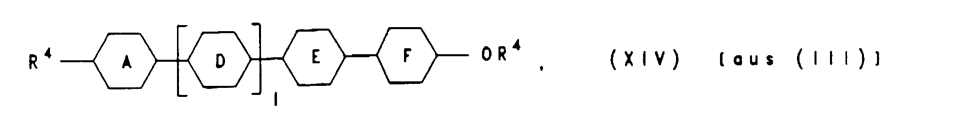 worin die Symbole die in Formel (III) angegebenen Bedeutungen haben.

Analoge Umsetzungen lassen sich mit (XI), (XII) und (XIII) vornehmen.

Ferner können durch Reaktion mit Carbonsäuren bzw. Carbonsäurederivaten (z.B. Halogenide oder Anhydride) Carbonsäurearylester erhalten werden, z.B. (XV) aus (XI), 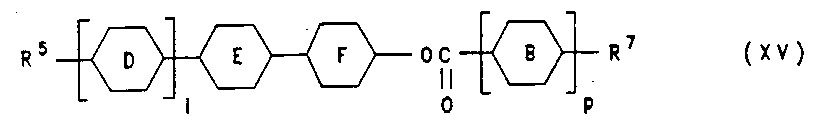 worin die Symbole die in Formel (I) und (VIII) angegebenen Bedeutungen haben und p Null oder Eins oder Zwei sein kann.

Analoge Umsetzungen lassen sich mit (X), (XII) und (XIII) durchführen.

Ferner können durch Umsetzung von Perfluoralkansulfonsäurederivaten mit (IX), (X), (XI), (XII) oder (XIII) Perfluoralkansulfonsäureester-Zwischenprodukte erhalten werden, die nach Standardmethoden, wie z.B. in DE-C 3 930 663, EP-A 354 434 und der deutschen Patentanmeldung P 42 36 103.6 mit dem Titel "Verfahren zur Kreuzkupplung von aromatischen Boronsäuren mit aromatischen Halogenverbindungen oder Perfluoralkylsulfonaten" beschrieben, mit Boronsäuren unter Metallkatalyse gekuppelt werden zu Komponenten von Flüssigkristallen der Formel (XVI bis XIX):
worin 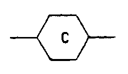 1,4-Phenylen, das auch 1, 2 oder 3 F-Substituenten tragen kann; Pyridin-2,5-diyl und Naphthalin-2,6-diyl bedeutet.

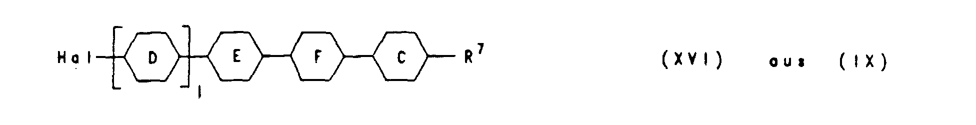

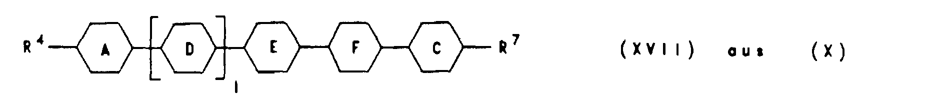

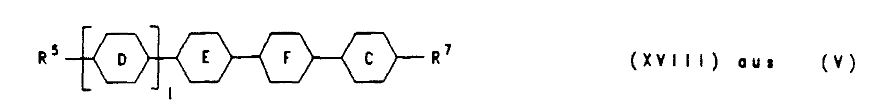

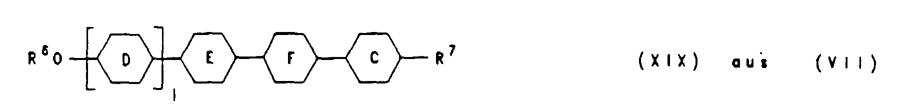

Bevorzugt lassen sich die erfindungsgemäßen Verbindungen der Formel (I) auf eine der oben beschriebenen Weisen zur Herstellung von Komponenten von Flüssigkristallen der Formel (XX) verwenden; worin
- r: 0, 1, 2 oder 3 bedeutet und
- R⁴: die in Formel (II) angegebene Bedeutung hat:

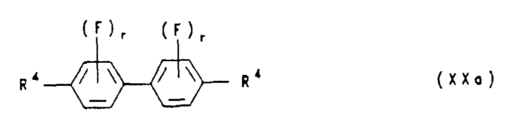

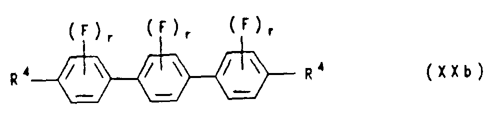

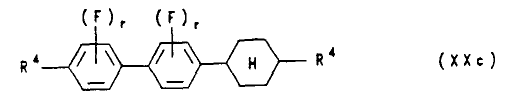

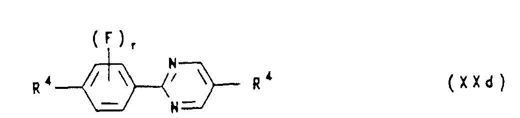

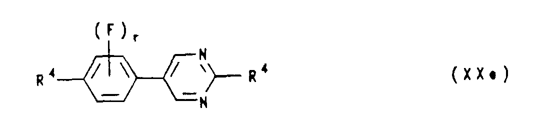

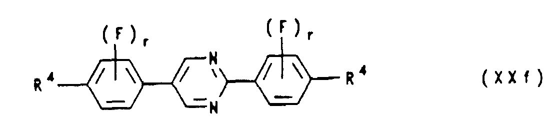

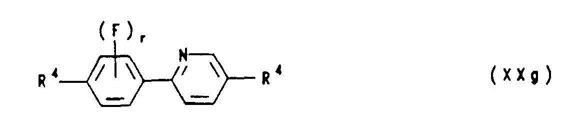

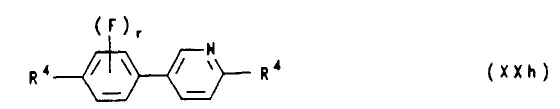

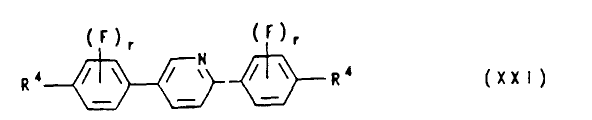

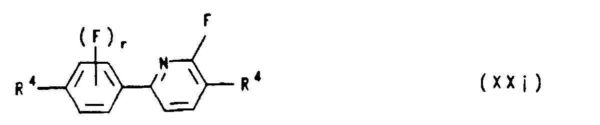

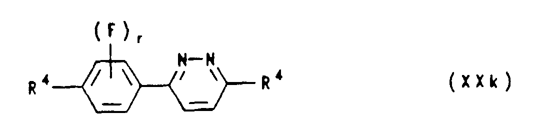

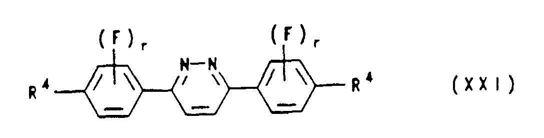

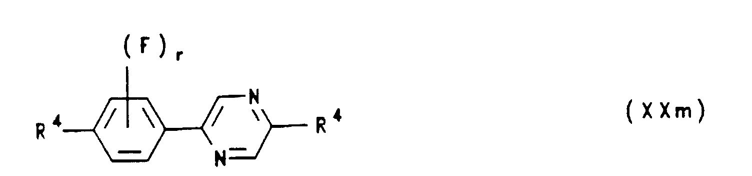

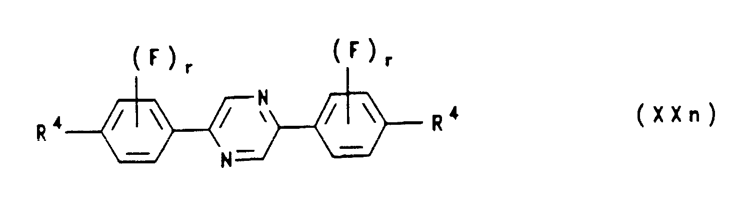

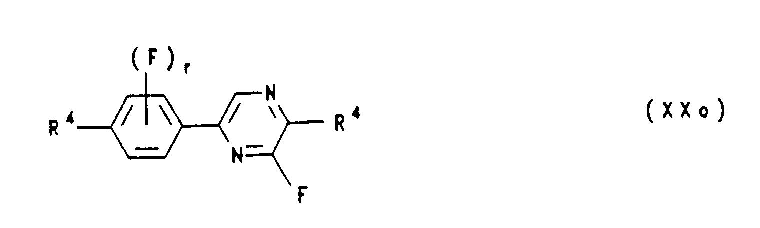

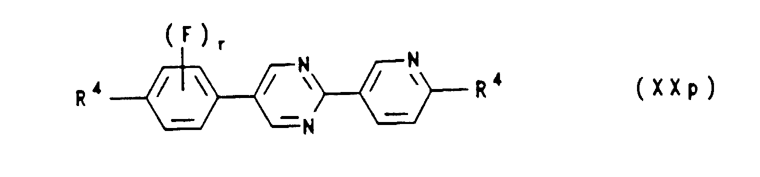

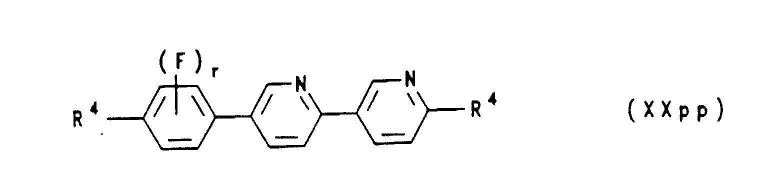

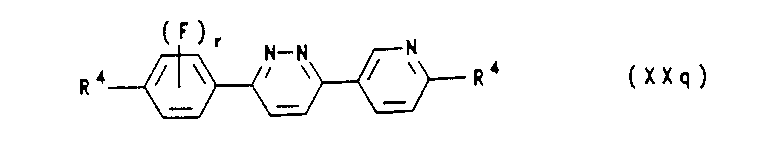

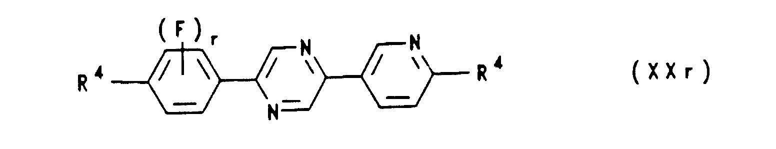

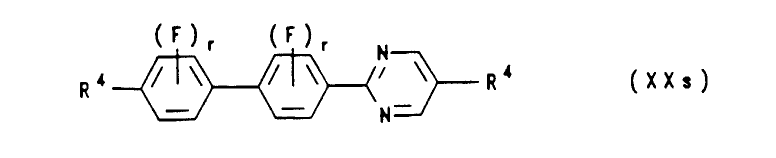

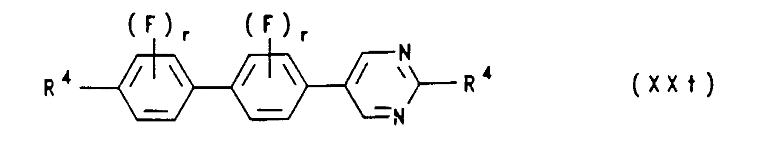

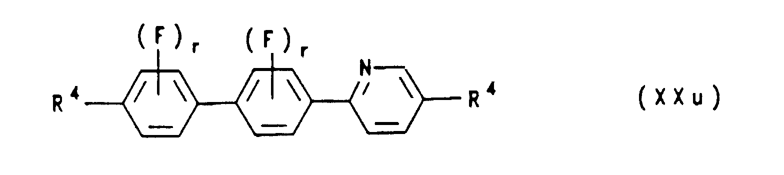

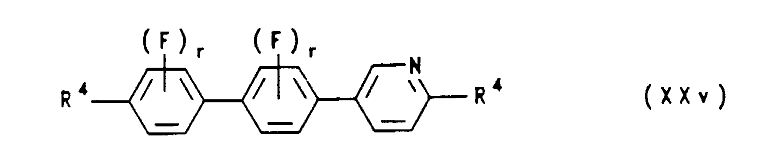

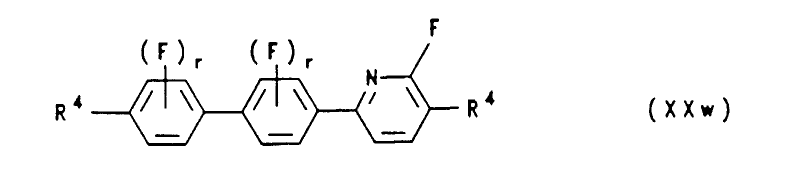

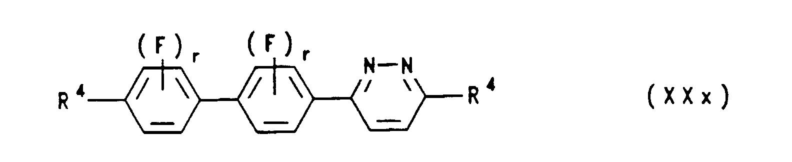

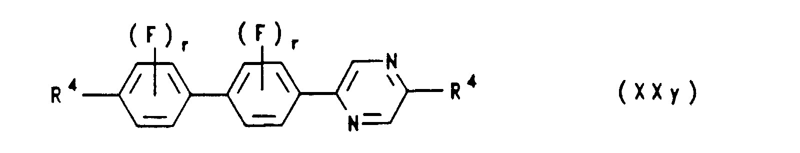

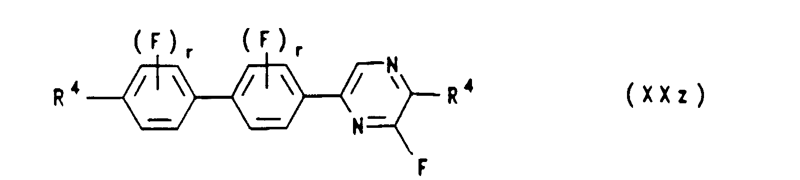

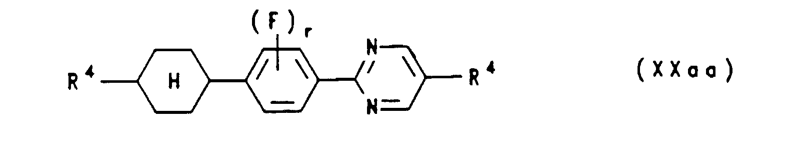

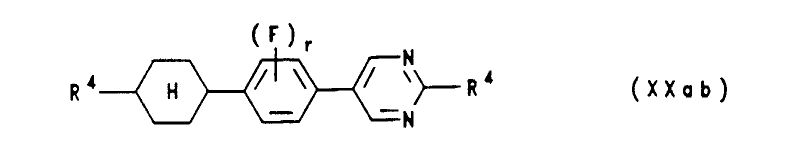

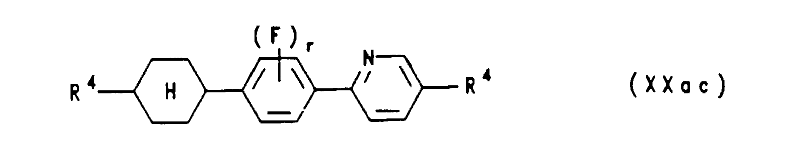

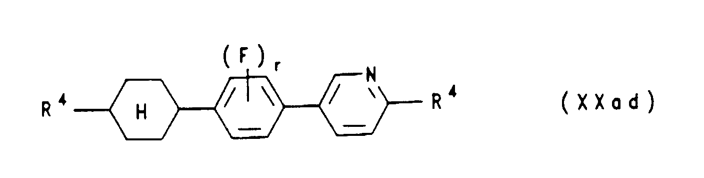

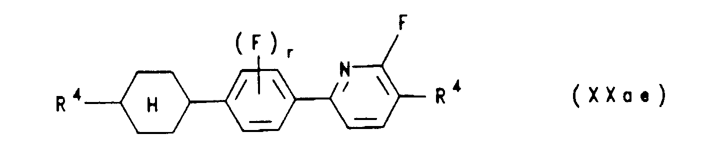

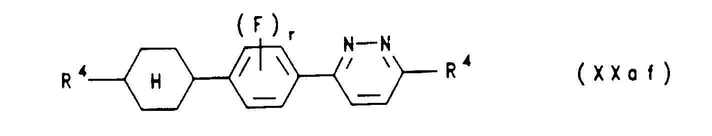

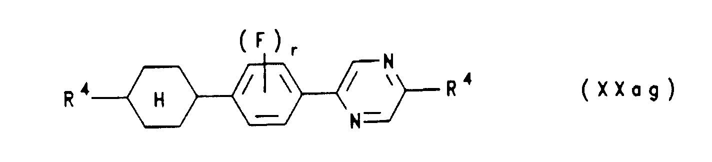

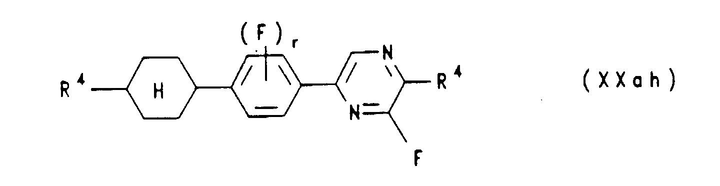

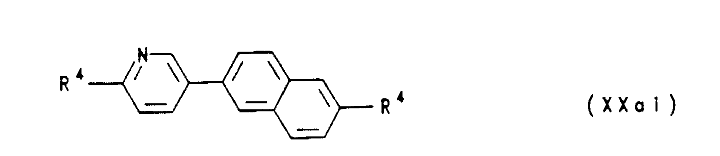

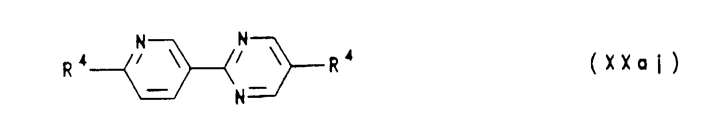

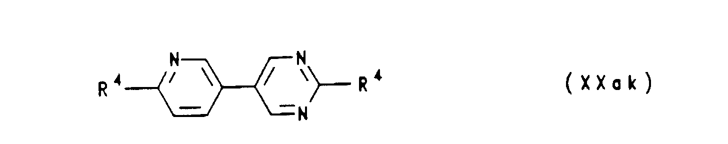

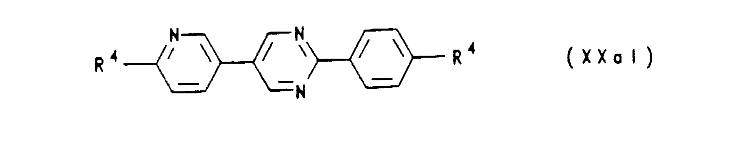

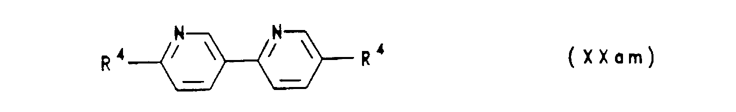

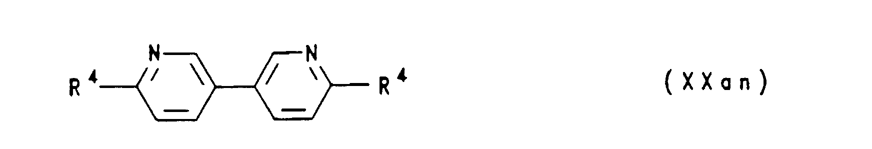

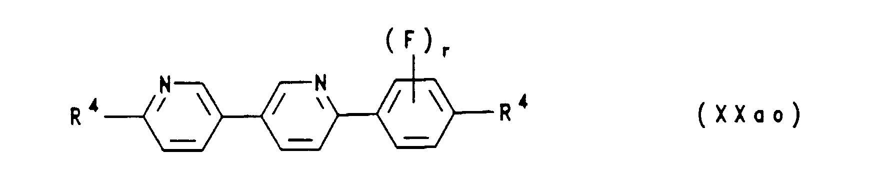

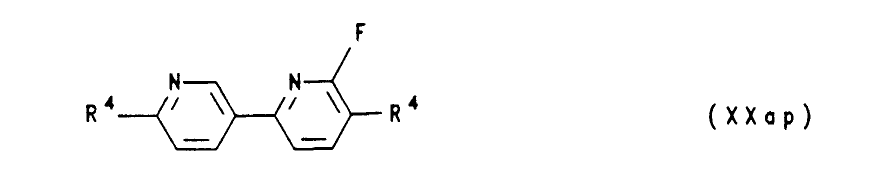

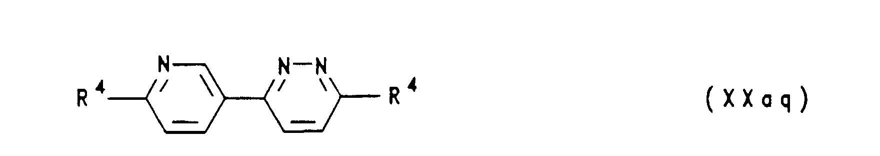

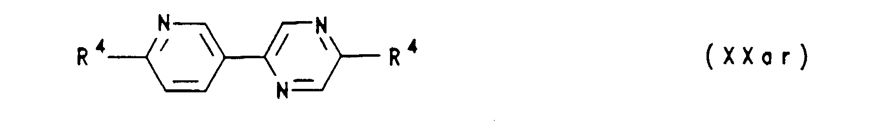

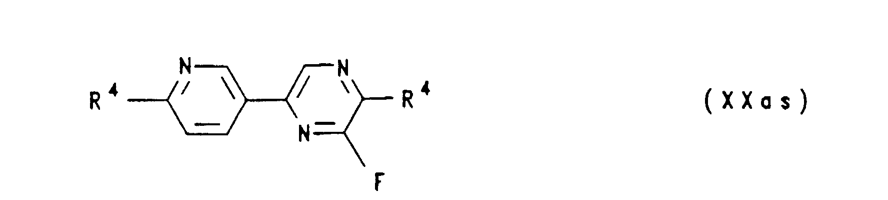

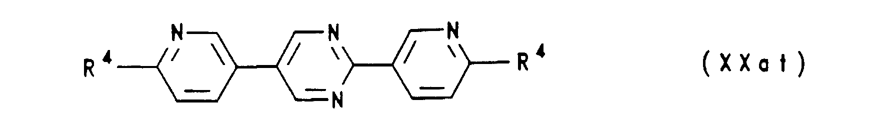

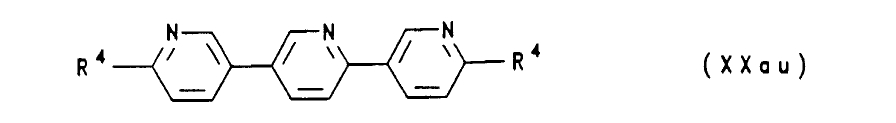

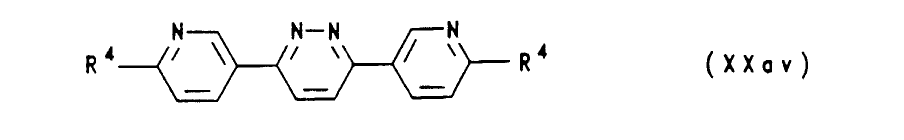

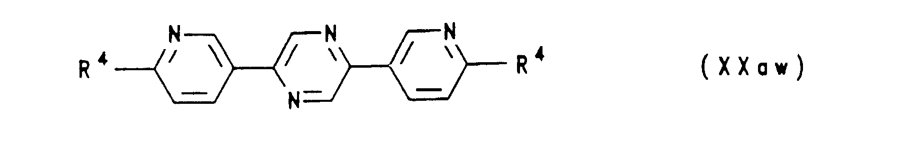

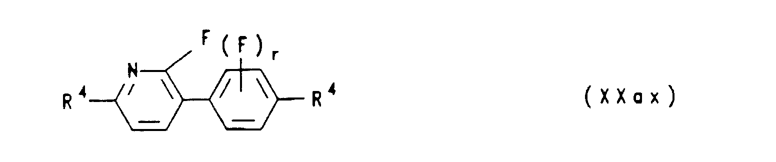

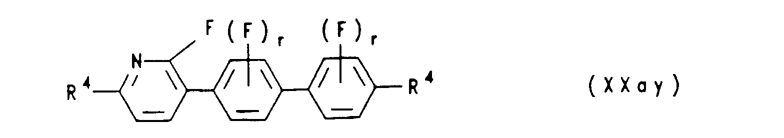

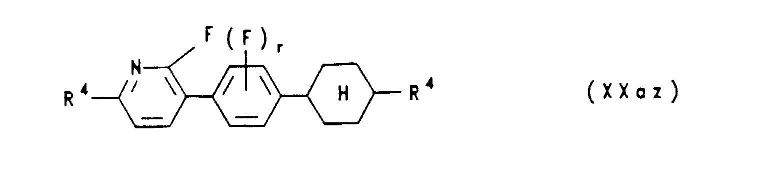

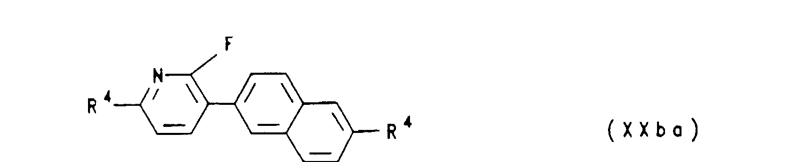

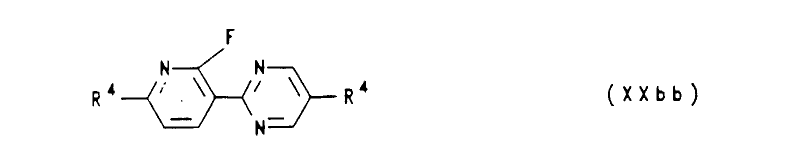

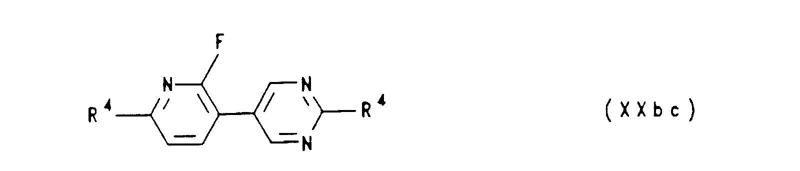

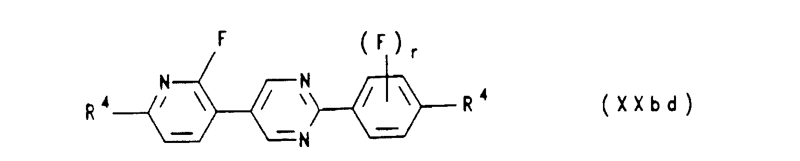

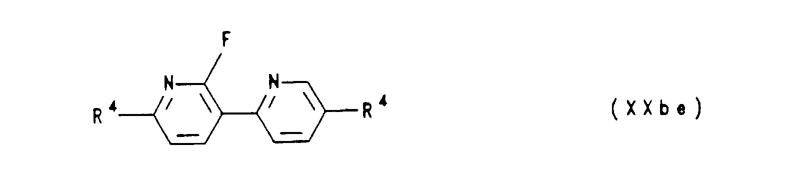

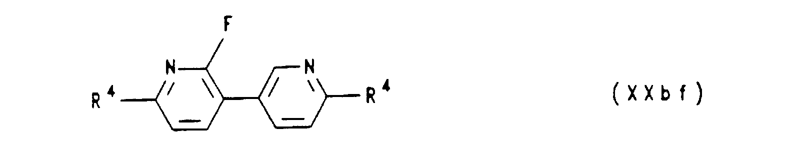

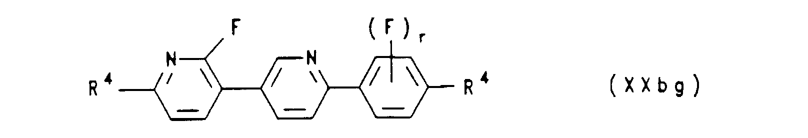

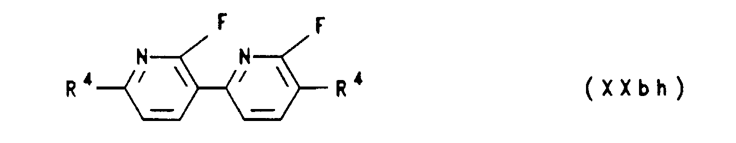

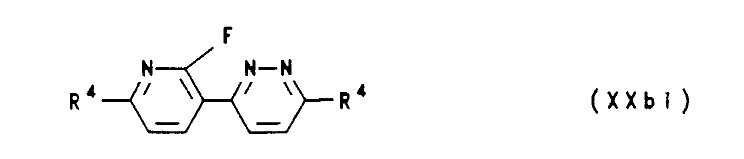

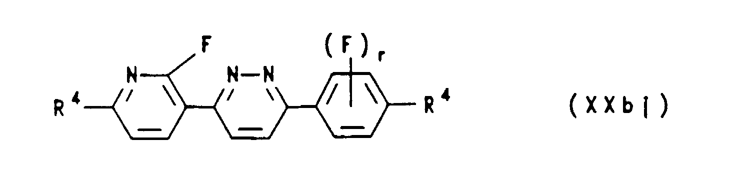

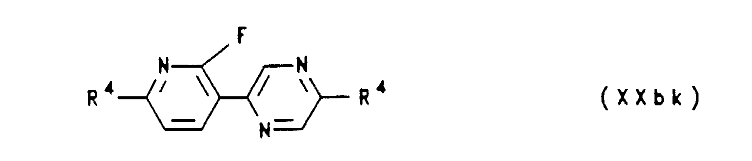

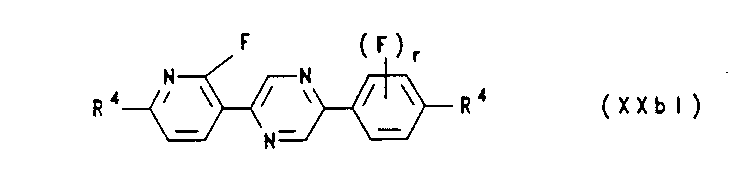

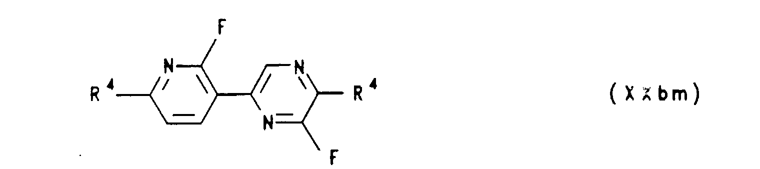

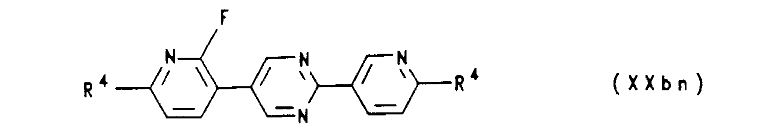

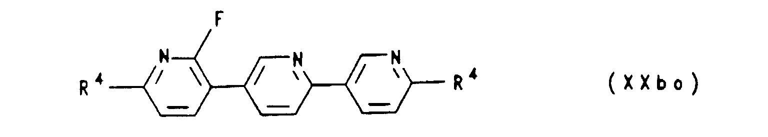

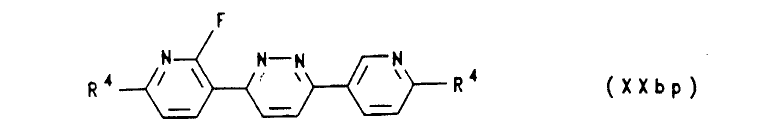

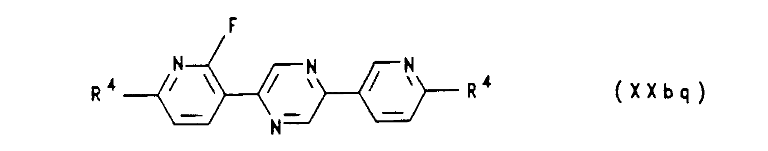

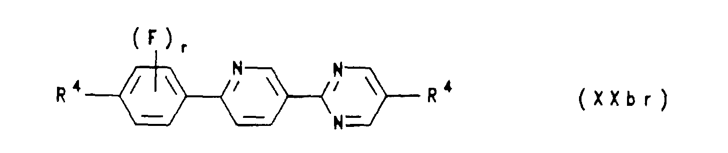

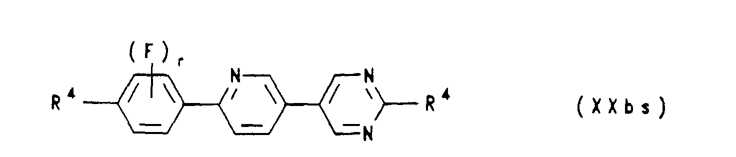

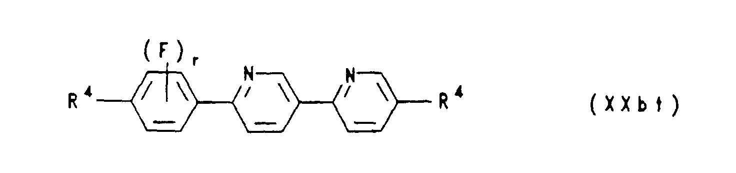

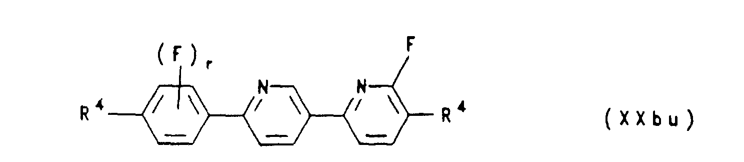

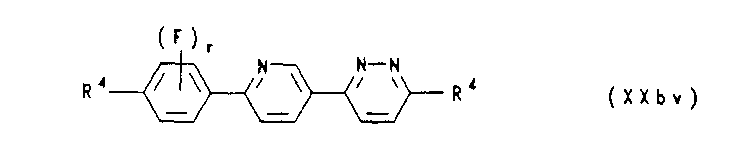

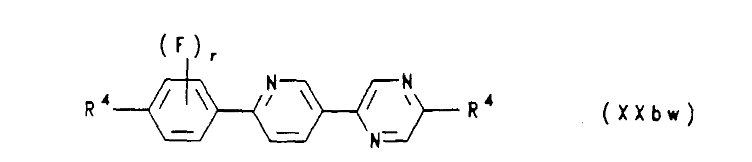

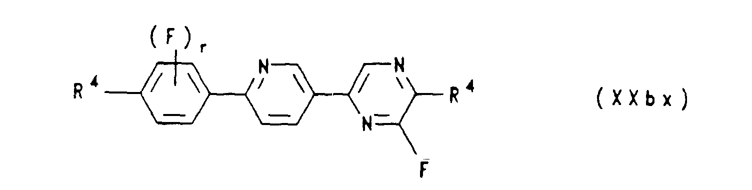

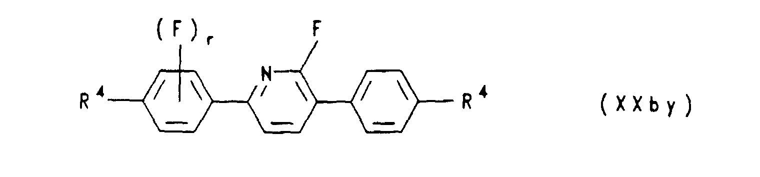

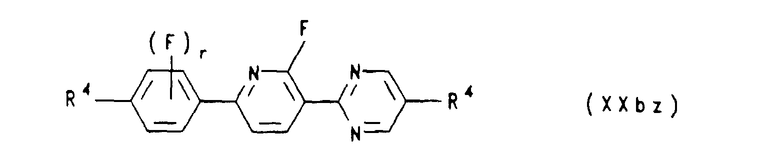

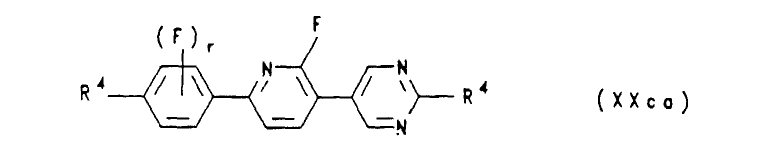

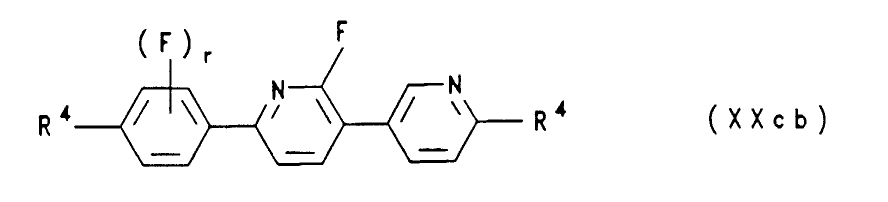

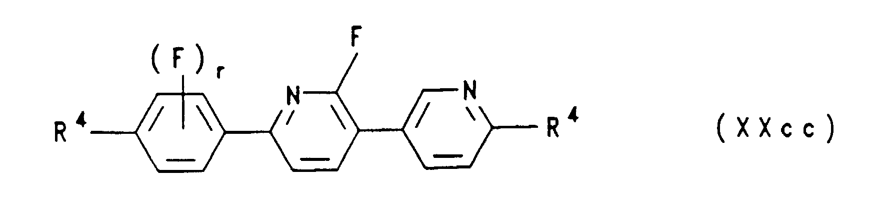

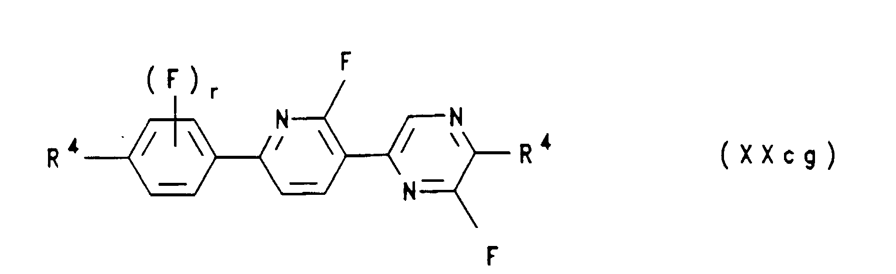

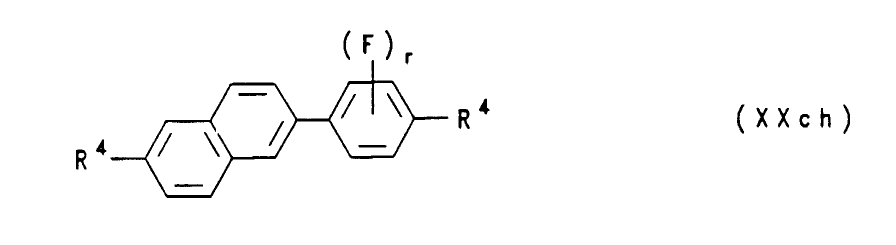

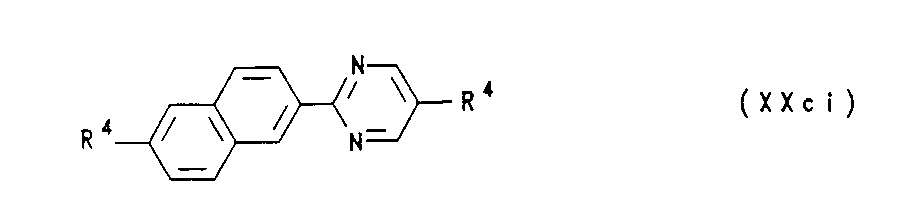

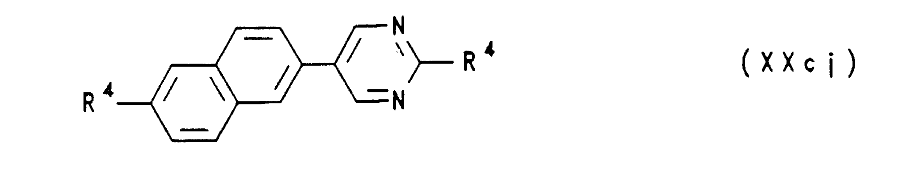

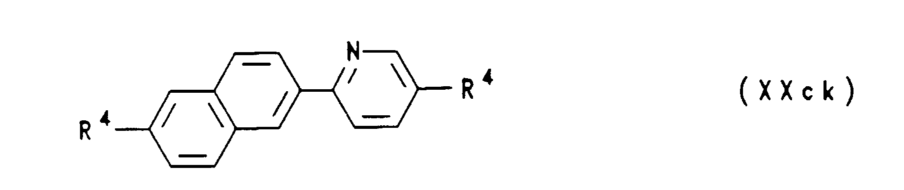

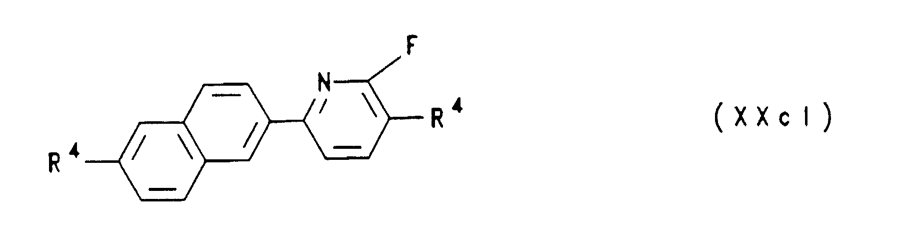

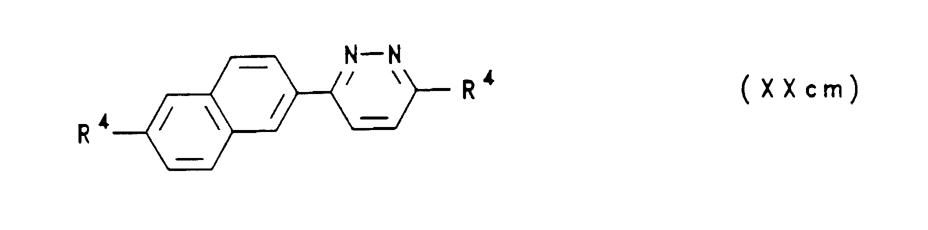

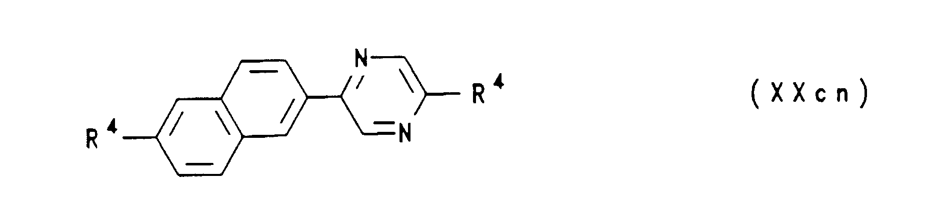

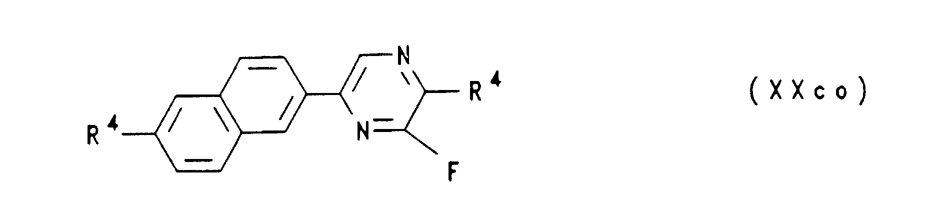

Die Herstellung der Verbindungen (I) kann besonders vorteilhaft dadurch erfolgen, daß eine Arylboronsäure der Formel (XXI), wie sie in der deutschen Patentanmeldung P 42 36 105.2 mit dem Titel "Arylboronsäuren als Vorprodukte zur Herstellung von Komponenten von Flüssigkristallen" vorgeschlagen wird: 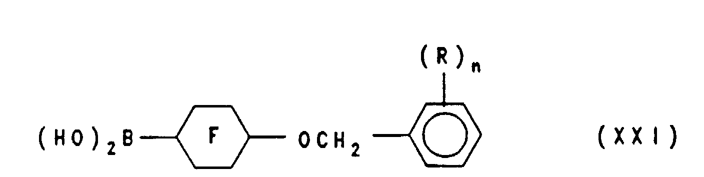 in der die Substituenten und Indizes die folgende Bedeutung aufweisen: 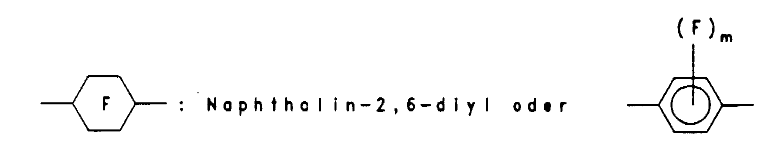
- R:: CH₃, OCH₃
- m:: 0, 1, 2 oder 3
- n:: 0, 1 oder 2
nach bekannten Verfahren (z.B. EP-A 354 434 oder wie in der deutschen Patentanmeldung P 42 36 103.6 mit dem Titel "Verfahren zur Kreuzkupplung von aromatischen Boronsäuren mit aromatischen Halogeniden bzw. - Perfluoralkylsulfonaten" vorgeschlagen) mit einem Halogenid der Formel (XXII) gekuppelt wird: 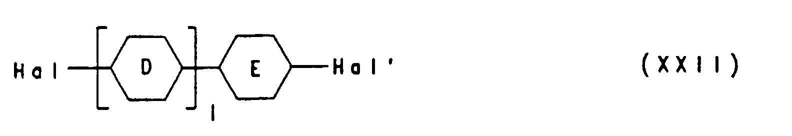 worin die Symbole die in Formel (I) angegebenen Bedeutungen haben und Hal': Cl, Br, I und Perfluoralkansulfonat bedeutet.

Bevorzugt werden die Verbindungen

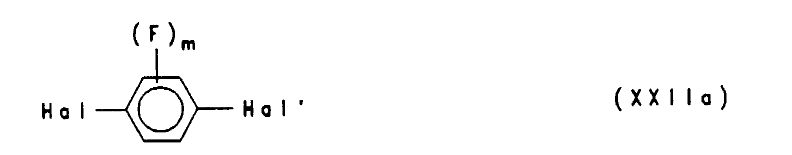

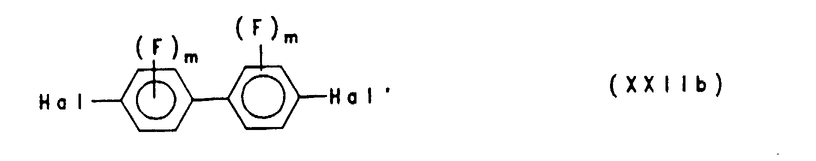

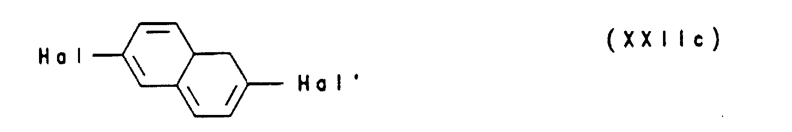

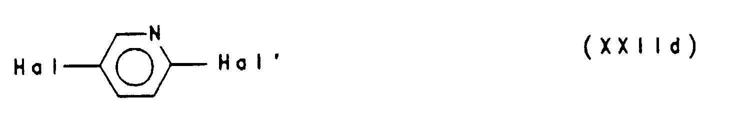

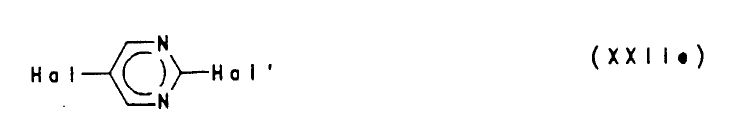

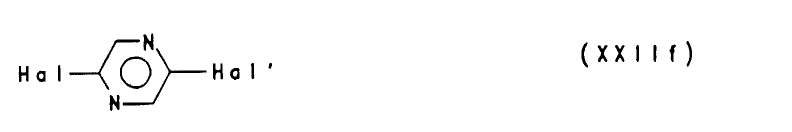

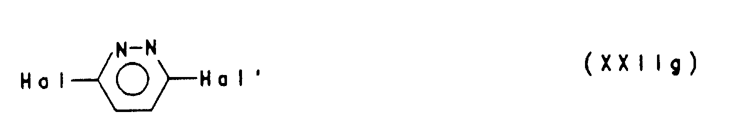

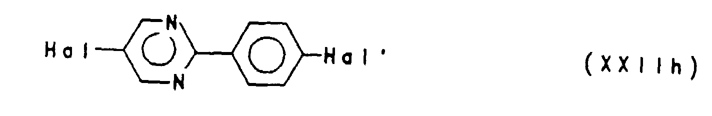

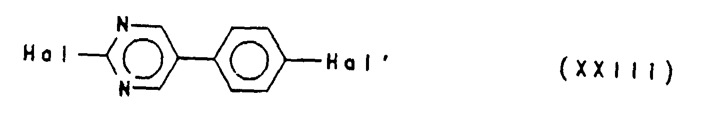

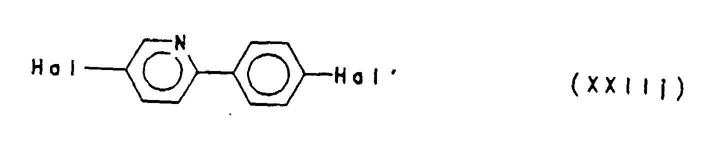

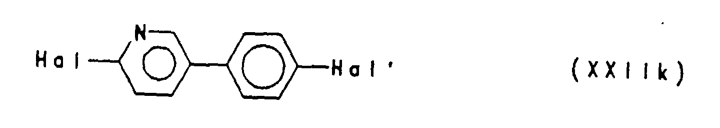

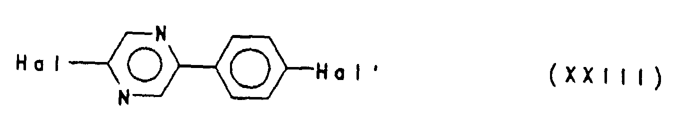

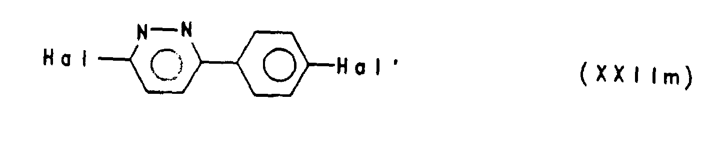

Sie sind entweder im Handel erhältlich, z.B. (XXIIa; m=0), (XXIId), (XXIIg) oder lassen sich nach Standardmethoden aus bekannten bzw. im Handel erhältlichen Materialien erhalten: z.B. (XXIIb, m=0) durch Umsetzung von käuflichem 4-Brom-4'-hydroxy-biphenyl mit Perfluoralkansulfonsäurederivaten; z.B. (XXllc) durch Umsetzung von käuflichen 6-Brom-2-hydroxy-naphthalin mit Perfluoralkansulfonsäurederivaten; (XXIIe) wie in J.Chem.Soc. (C) 1971, 1889 beschrieben, (XXllm) in Analogie zu Z.Chem. 17, 333 (1977); (XXIII) durch Umsetzung von 2-(4-Halophenyl)-5-hydroxy-pyrazin- hergestellt analog H. Heberer, Diplomarbeit Halle, 1967, zitiert in "Flüssige Kristalle in Tabellen", Hrsg. D.Demus, VEB Verlag für Grundstoffindustrie, Leipzig 1974, p. 265 - mit z. B. Phosphorhalogeniden oder Perfluoralkansulfonsäurederivaten; (XXIIk) analog (XXIII), jedoch unter Verwendung von 5-(4-Halophenyl)-2-hydroxypyridin, hergestellt analog zu Z.Chem. 18, 403 (1978); (XXIIi) analog (XXIII), jedoch unter Verwendung von 5-(4-Halophenyl)-2-hydroxy-pyrimidin, hergestellt analog J.Prakt. Chem. 321, 169 (1979); (XXIIh) analog Mol.Cryst.Liq.Cryst. 42, 1225 (1977); (XXllf) wie in J.Am.Chem.Soc. 71, 2798(1949) beschrieben; (XXIII) analog zu "Adv. in Liquid Crystal Research and Application (ed.L.Bata), Oxford, Pergamon Press, Budapest".

Die erfindungsgemäßen Verbindungen sind vielseitige Synthesebausteine zur Darstellung mehrkerniger aromatischer Verbindungen, die sich in vielen Bereichen der organischen Chemie, beispielsweise zur Darstellung von Komponenten für Flüssigkristallmischungen, Pharmaka, Kosmetika oder Pflanzenschutzmittel, einsetzen lassen.

Bevorzugt ist die Verwendung als Zwischenprodukte für die Darstellung von Komponenten für Flüssigkristallmischungen, insbesondere ferroelektrische. Solche Komponenten sind z.B. in EP-A 354 434, EP-A 307 880, EP-B 283 326, EP-B 357 702 und EP-A 439 089 beschrieben.

Durch Einsatz der erfindungsgemäßen Verbindungen der Formel (I) können bei der Herstellung von Komponenten für Flüssigkristallmischungen Synthesestufen eingespart werden, was, insbesondere in der großtechnischen Synthese, enorme Vorteile bringt. Die Erfindung erlaubt die Bereitstellung einer großen Produktpalette aus jeweils einem Zwischenprodukt, was die Prozeßführung in technischer, ökonomischer und ökologischer Sicht stark vereinfacht.

Ein Vergleich der Schemata 1A - für ein Verfahren unter Verwendung von Verbindungen gemäß EP-A 354 434 - und 1 B - für ein Verfahren unter Verwendung der erfindungsgemäßen Verbindungen - offenbart, daß für die Synthese von 2 homologen Phenylpyridimidinen nach dem Verfahren mit den Verbindungen gemäß EP 354 434 insgesamt 8 Synthesestufen erforderlich sind, ein Verfahren unter Nutzung der erfindungsgemäßen Verbindungen der Formel (I) jedoch nur 7 Synthesestufen für die Herstellung derselben zwei Homologe benötigt.

Noch deutlicher gestaltet sich der Vorteil für Prozeßökonomie und -ökologie, wenn - wie z.B. in EP-A 307 880, Bspl. 34 beschrieben - 3 Homologe oder - wie z.B. in EP-B 283 326, Bsp. 5, - 4 Homologe einer Substanzklasse hergestellt werden müssen.

Schema 2 A zeigt eine Synthese mit den in EP-A 354 434 vorgeschlagenen Verbindungen. Für die Herstellung der drei homologen Phenylpyrimidine werden, unter Einsatz der angegebenen Vorprodukte, 12 Synthesestufen benötigt. Schema 2 B gibt die Synthese derselben 3 Homologe, jedoch unter Verwendung des erfindungsgemäßen bifunktionellen Vorproduktes (Ib), wieder; es sind nur 8 Synthesestufen erforderlich.

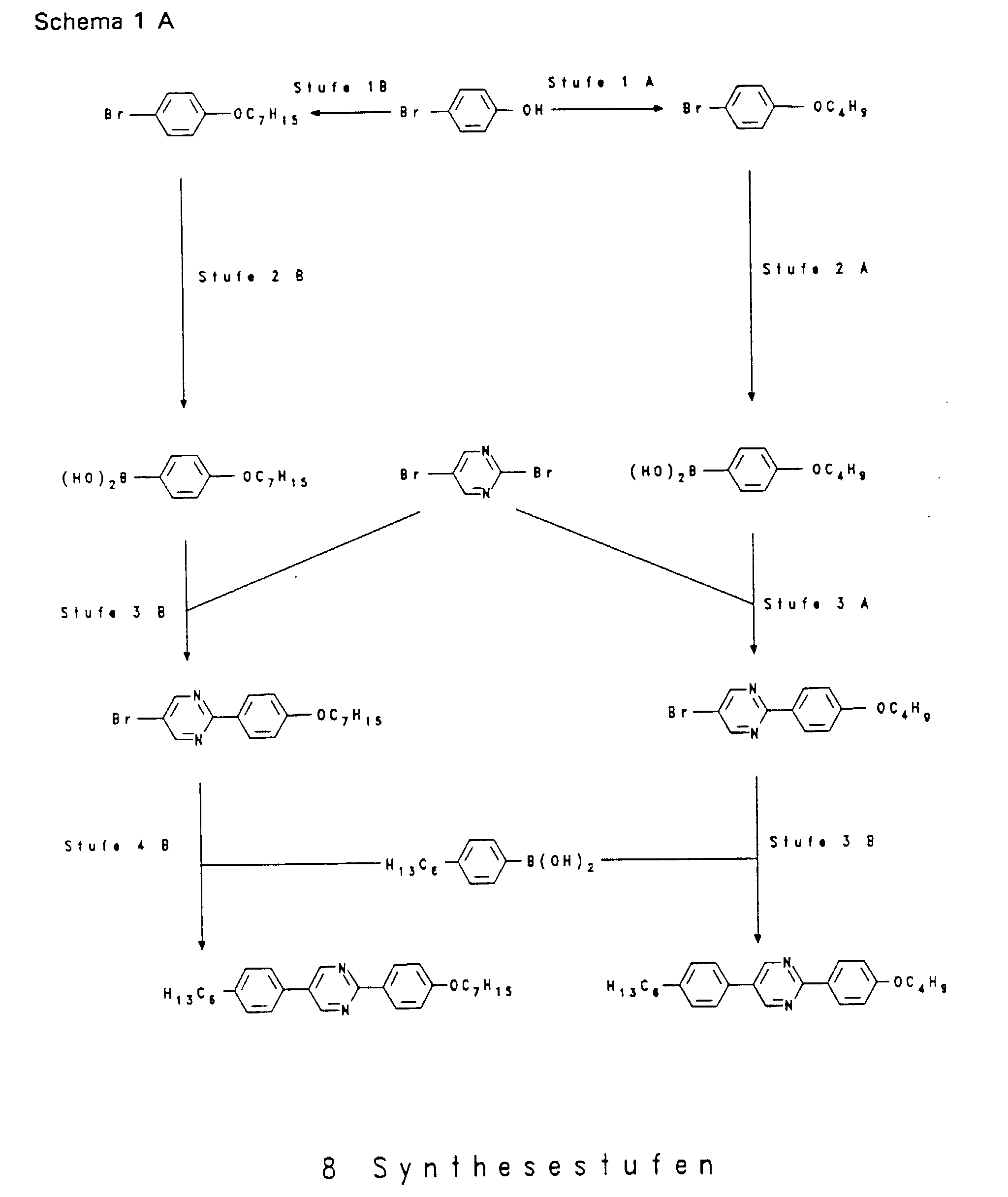

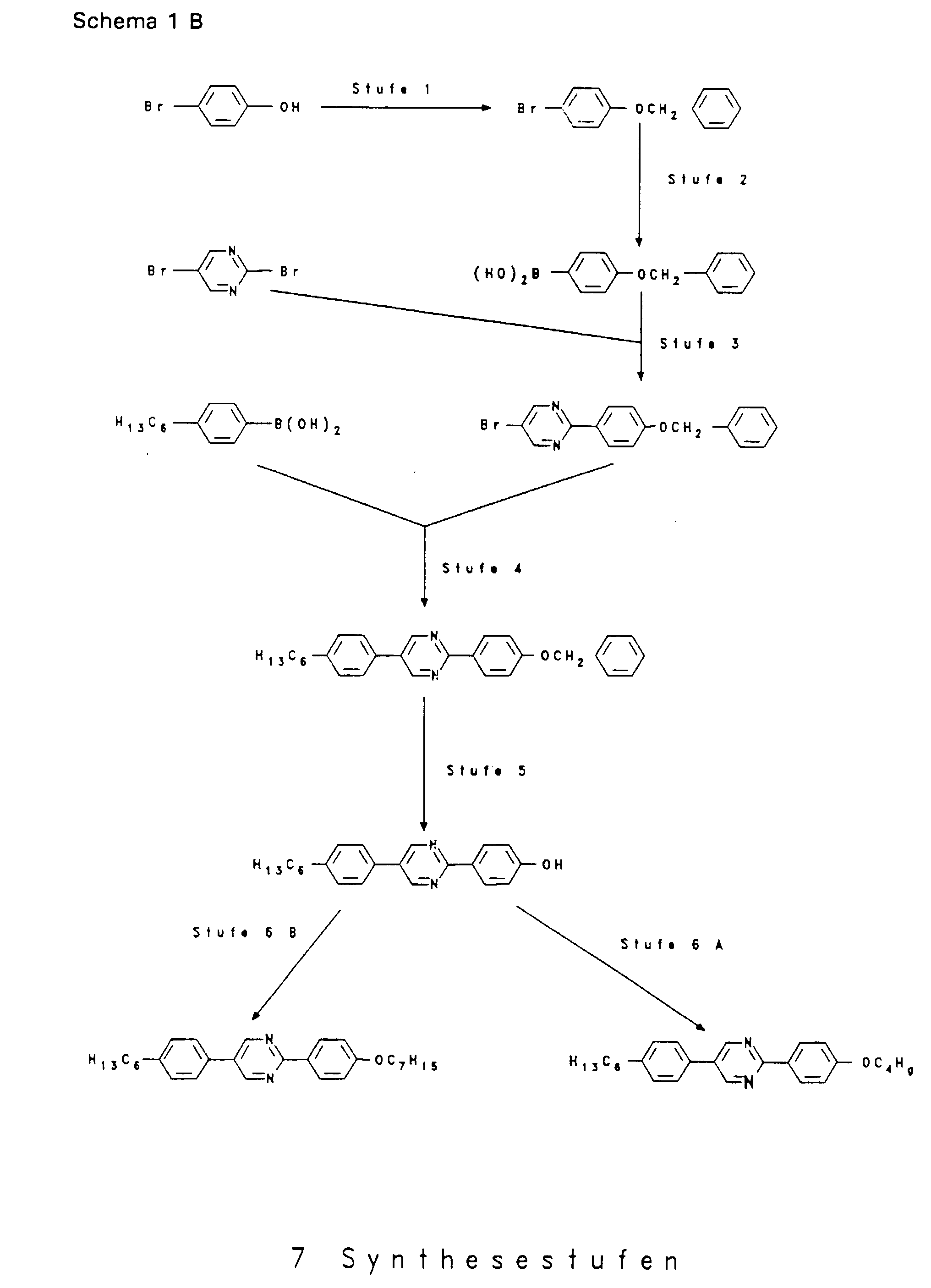

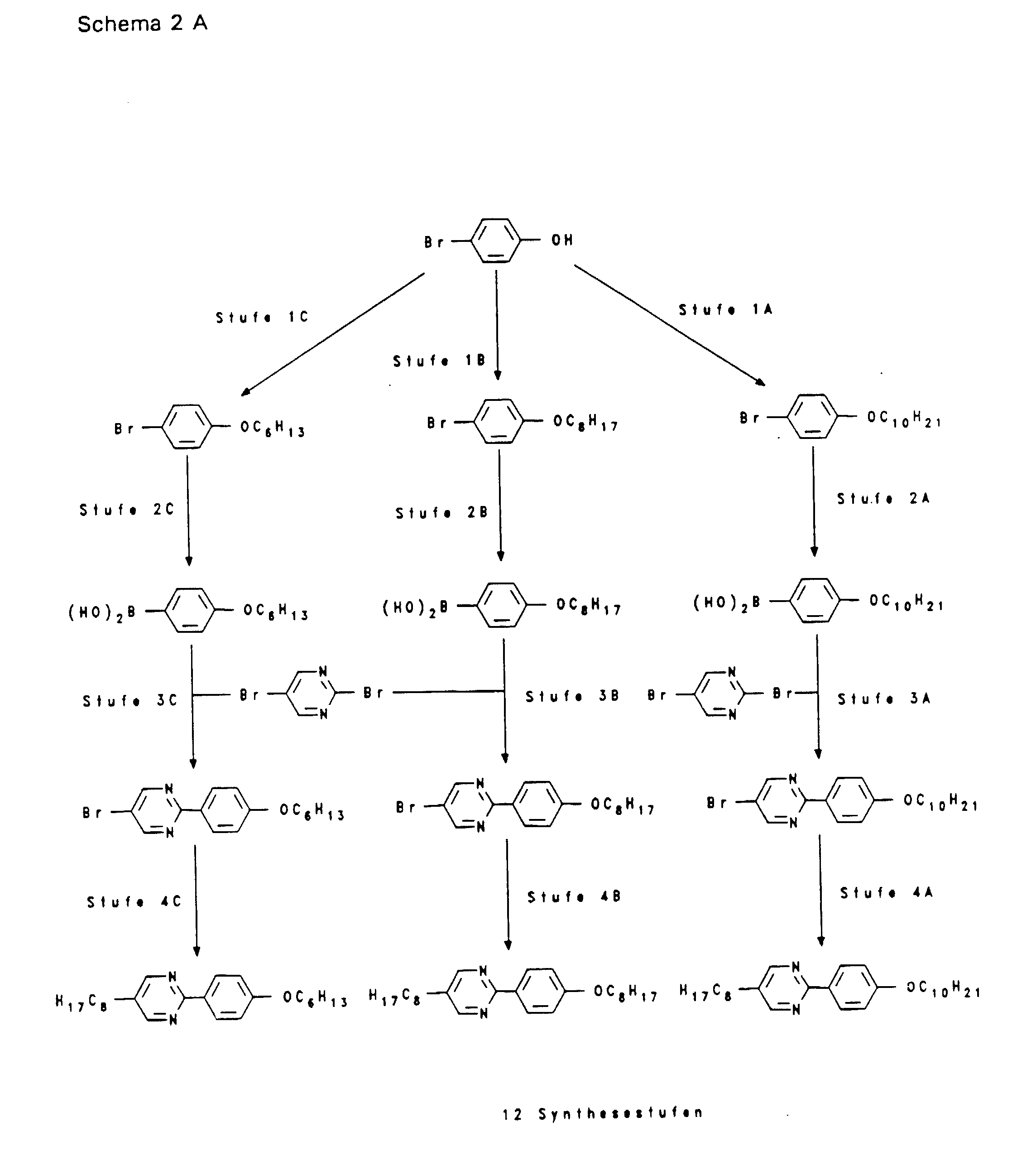

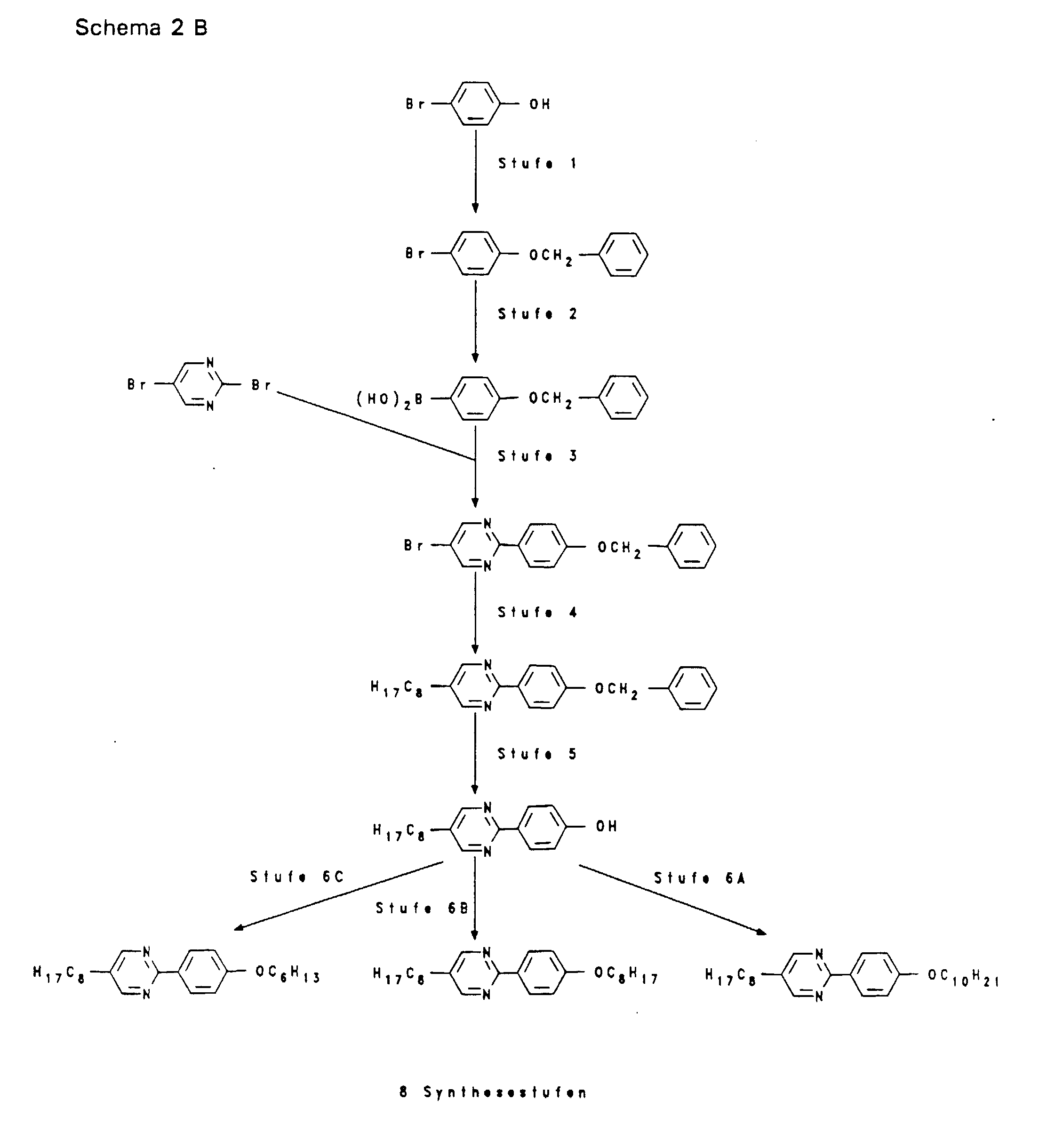

Die ausgezeichnete Eignung der Verbindungen (I) als universell einsetzbare Vorprodukte, z.B. für Flüssigkristalle, wird ferner durch Schema 3 belegt. In EP-A 328 330 wird eine Flüssigkristallverbindung vorgeschlagen, die aus zwei Typen von Phenylpyrimidinen besteht, von denen jeder Typ mit 3 Homologen in der Mischung (Tab.4, Bspl 14) vertreten ist:
- Typ A:: 2-(4-Alkoxy-phenyl)-5-alkyl-pyrimidine
- Typ B:: 2-(4-Alkyl-biphenyl-4'-yl)-5-alkyl-pyrimidine
In Schema 3 ist dargestellt, daß die erfindungsgemäße Verbindung (1b) zur Herstellung beider Typen geeignet ist. Dies ermöglicht die Herstellung einer größeren Menge an (Ib), was sowohl hinsichtlich Prozeß-ökonomie als auch Prozeß-ökologie einen deutlichen Vorteil darstellt, verglichen mit der jeweils in kleinen Produktionsvolumina erfolgenden linearen Synthese für jeden der Typen A bzw. B.

Schema 4 belegt, daß auch eine Mischung, die neben obigem Phenylpyrimidin Typ A noch zwei weitere Typen,
- C:: 5-Alkyloxy-2-[4-substituiertes Phenyl]pyrimidin
- D:: 5-(4-substituiertes Phenyl)-2-(4-substituiertes Phenyl)pyrimidin
enthält und in EP-A 469 800, Tab.2, Bsp.9 vorgeschlagen wird, sich günstig aus der erfindungsgemäßen Verbindung (Ib) herstellen läßt.

In ihrer Gesamtheit belegen die Schemate 1 bis 4, daß sich die bifunktionellen Vorprodukte der Formel (I) zur Herstellung verschiedenster Typen von Flüssigkristallen aus 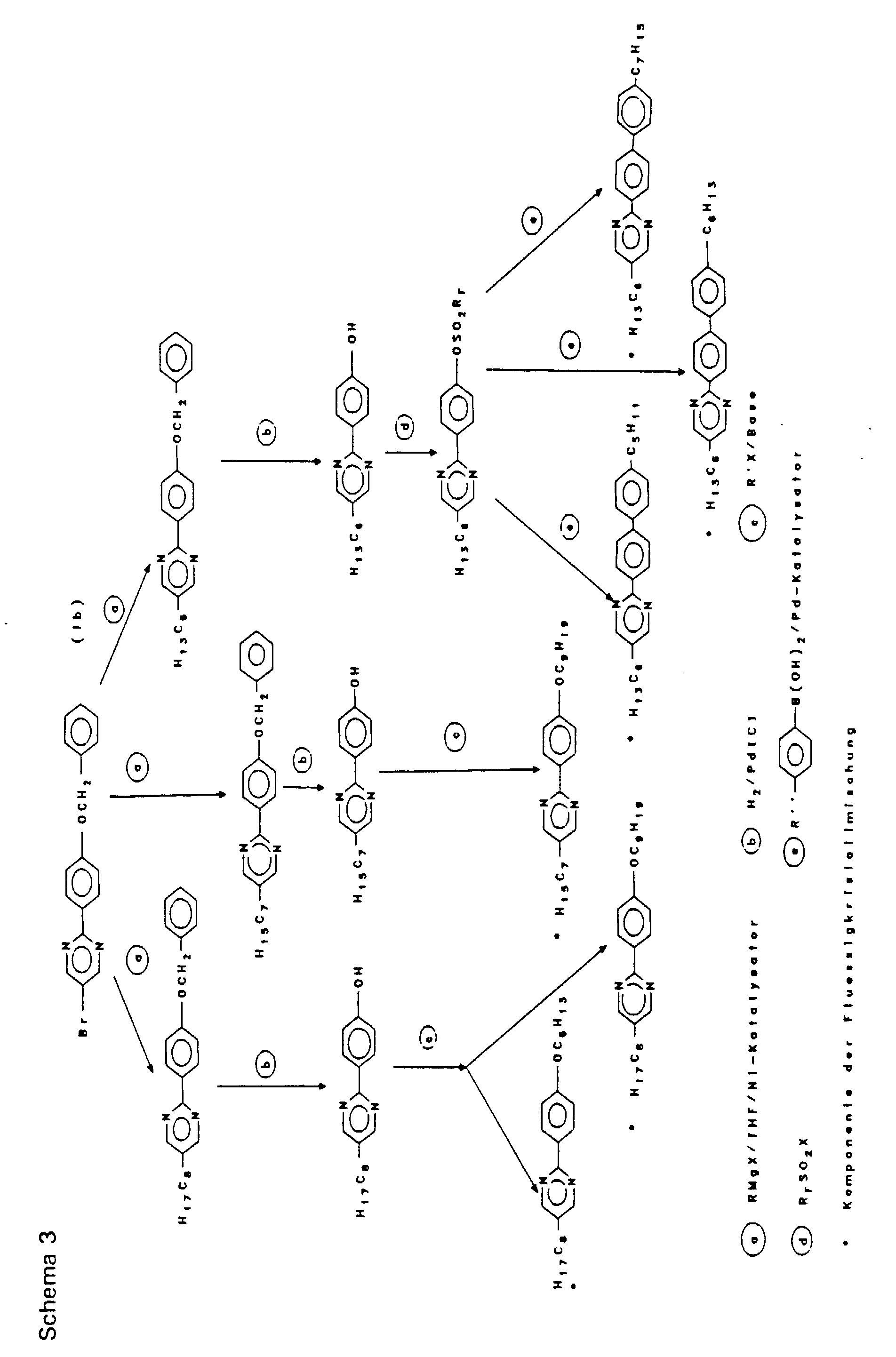 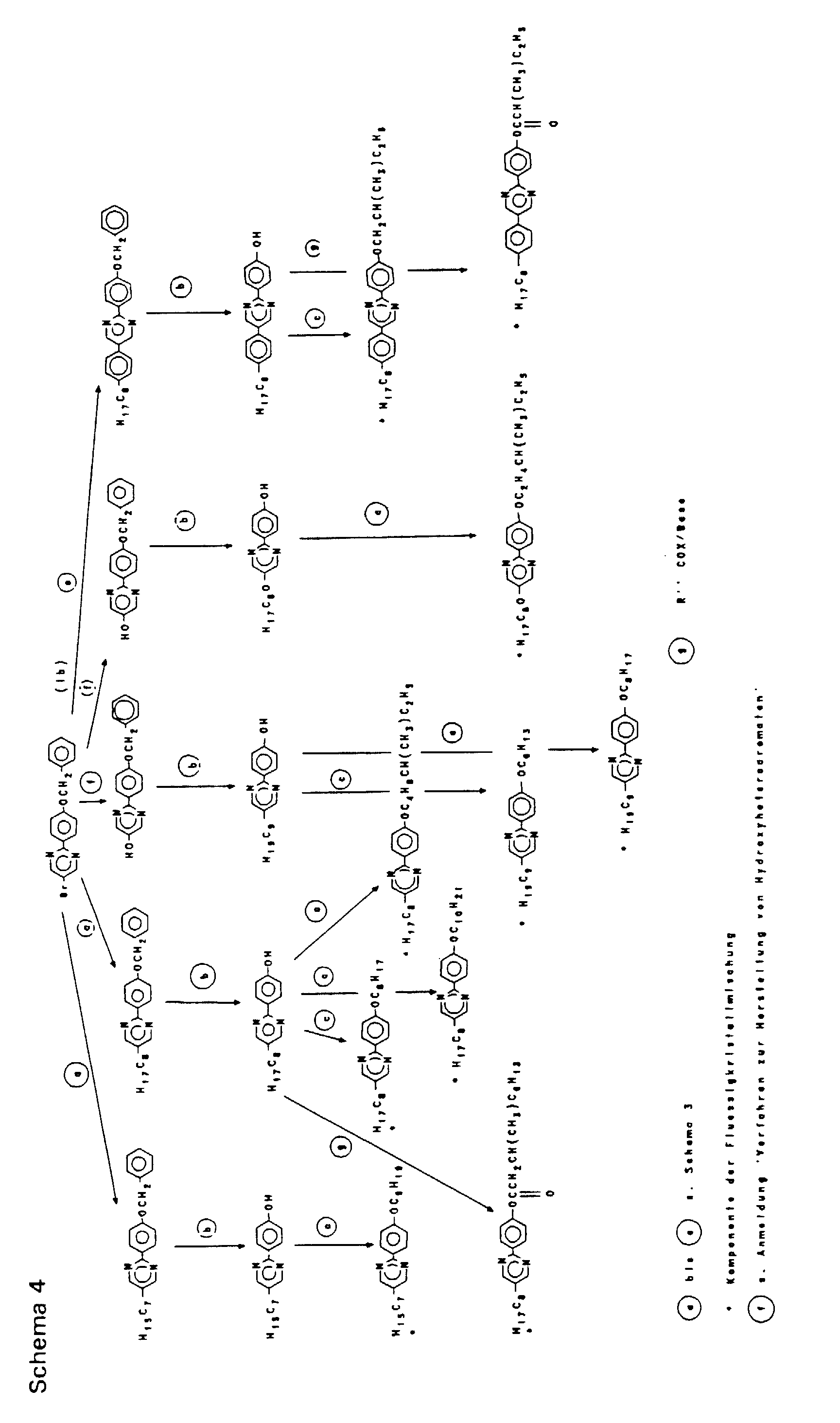 einem gemeinsamen Vorprodukt bestens eignen. Die Herstellung eines derart universellen Vorproduktes und in Folge der daraus abgeleiteten Endprodukte kann wesentlich ökonomischer erfolgen als die mehrerer einzelner Vorprodukte.

### Beispiel 1

5-Brom-2-[4-(benzyloxy)phenyl]pyrimidin

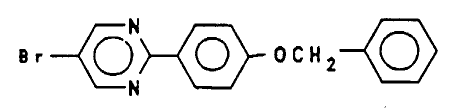

Eine Lösung von 104 g 2,5-Dibrompyrimidin, 100 g 4-Benzyloxyphenylboronsäure,
4.75 g Pd (10% auf Aktivkohle), 4.5 g Triphenylphosphan und 93 g Natriumcarbonat in 1 l Toluol, 0.5 l Ethanol und 0.3 l Wasser wird für 24 h auf 80°C erhitzt. Nach Filtration wird die organische Phase abgetrennt und im Vakuum zur Trockne gebracht. Der Rückstand wird aus Acetonitril umkristallisiert: 83 g Feststoff v. Schmp. 153-155°C.

Analog werden die Beispiel 2-37 erhalten
- Bsp. 2: 5-Brom-2-[(4-benzyloxy-2,3-difluor)phenyl]-pyrimidin
- Bsp. 3: 5-Brom-2-[(4-benzyloxy-3-fluor)phenyl]-pyrimidin
- Bsp. 4: 5-Brom-2-[(4-benzyloxy-2-fluor)phenyl]-pyrimidin
- Bsp. 5: 5-Chlor-2-[(4-benzyloxy)phenyl]-pyrimidin
- Bsp. 6: 5-Chlor-2-[(4-benzyloxy-2,3-difluor)phenyl]-pyrimidin
- Bsp. 7: 5-Chlor-2-[(4-benzyloxy-3-fluor)phenyl]-pyrimidin
- Bsp. 8: 5-Chlor-2-[(4-benzyloxy-2-fluor)phenyl]-pyrimidin
- Bsp. 9: 5-Jod-2-[(4-benzyloxy)phenyl]-pyrimidin
- Bsp. 10: 5-Jod-2-[(4-benzyloxy-2,3-difluor)phenyl]-pyrimidin
- Bsp. 11: 5-Jod-2-[(4-benzyloxy-3-fluor)phenyl]-pyrimidin
- Bsp. 12: 5-Jod-2-[(4-benzyloxy-2-fluor)phenyl]-pyrimidin
- Bsp. 13: entfällt
- Bsp. 14: 5-Brom-2-[4-(benzyloxy)phenyl]pyridin; Schmp. 159-160°C
- Bsp. 15: 5-Brom-2-[(4-benzyloxy-2,3-difluor)phenyl]-pyridin
- Bsp. 16: 5-Brom-2-[(4-benzyloxy-3-fluor)phenyl]-pyridin
- Bsp. 17: 5-Brom-2-[(4-benzyloxy-2-fluor)phenyl]-pyridin
- Bsp. 18: 5-Chlor-2-[(4-benzyloxy)phenyl]-pyridin
- Bsp. 19: 5-Chlor-2-[(4-benzyloxy-2,3-difluor)phenyl]-pyridin
- Bsp. 20: 5-Chlor-2-[(4-benzyloxy-3-fluor)phenyl]-pyridin
- Bsp. 21: 5-Chlor-2-[(4-benzyloxy-2-fluor)phenyl]-pyridin
- Bsp. 22: 5-Jod-2-[(4-benzyloxy)phenyl]-pyridin
- Bsp. 23: 5-Jod-2-[(4-benzyloxy-2,3-difluor)phenyl]-pyridin
- Bsp. 24: 5-Jod-2-[(4-benzyloxy-3-fluor)phenyl]-pyridin
- Bsp. 25: 5-Jod-2-[(4-benzyloxy-2-fluor)phenyl]-pyridin
- Bsp. 26: 5-Brom-2-[4-(benzyloxy)phenyl]pyrazin
- Bsp. 27: 5-Brom-2-[(4-benzyloxy-2,3-difluor)phenyl]-pyrazin
- Bsp. 28: 5-Brom-2-[(4-benzyloxy-3-fluor)phenyl]-pyrazin
- Bsp. 29: 5-Brom-2-[(4-benzyloxy-2-fluor)phenyl]-pyrazin
- Bsp. 30: 5-Chlor-2-[4-(benzyloxy)phenyl]pyrazin
- Bsp. 31: 5-Chlor-2-[(4-benzyloxy-2,3-difluor)phenyl]-pyrazin
- Bsp. 32: 5-Chlor-2-[(4-benzyloxy-3-fluor)phenyl]-pyrazin
- Bsp. 33: 5-Chlor-2-[(4-benzyloxy-2-fluor)phenyl]-pyrazin
- Bsp. 34: 5-Jod-2-[4-(benzyloxy)phenyl]pyrazin
- Bsp. 35: 5-Jod-2-[(4-benzyloxy-2,3-difluor)phenyl]-pyrazin
- Bsp. 36: 5-Jod-2-[(4-benzyloxy-3-fluor)phenyl]-pyrazin
- Bsp. 37: 5-Jod-2-[(4-benzyloxy-2-fluor)phenyl]-pyrazin
- Bsp. 38: 5-Brom-2-[5-benzyloxy-pyridin-2-yl]pyrimidin
- Bsp. 38 a: 6-Brom-3-(4-benzyloxy)phenyl-pyridazin
- Bsp. 38 b: 6-Chlor-3-(4-benzyloxy)phenyl-pyridazin; Schmp. 186-189°C
- Bsp. 38 c: 6-Jod-3-(4-benzyloxy)phenyl-pyridazin

### Beispiel 39

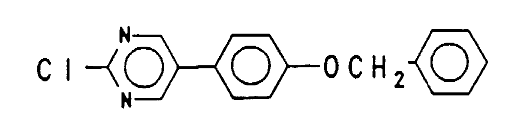

### 2-Chlor-5-[(4-benzyloxy)phenyl]pyrimidin

Eine Lösung von 3.87 g 5-Brom-2-chlor-pyrimidin, 4.56 g 4-Benzyloxyphenylboronsäure, 0.24 g Tetrakis(triphenylphosphin)-palladium (O) und 4.2 g Natriumcarbonat in 45 ml Toluol, 22 ml Ethanol und 15 ml Wasser wird für 2 h zum Sieden erhitzt. Aufarbeitung wie in Beispiel 1 und chromatographische Reinigung (SiO₂/CH₂Cl₂) ergeben 4.1 g farblosen Feststoff v. Schmp. 158-160°C.

Analog werden die Beispiel 40-54 erhalten:
- Bsp. 40: 2-Chlor-5-[(4-benzyloxy-2,3-difluor)phenyl]-pyrimidin
- Bsp. 41: 2-Chlor-5-[(4-benzyloxy-3-fluor)phenyl]-pyrimidin
- Bsp. 42: 2-Chlor-5-[(4-benzyloxy-2-fluor)phenyl]-pyrimidin
- Bsp. 43: 2-Brom-5-[(4-benzyloxy-2,3-difluor)phenyl]-pyrimidin
- Bsp. 44: 2-Brom-5-[(4-benzyloxy-2,3-difluor)phenyl]-pyrimidin
- Bsp. 45: 2-Brom-5-[(4-benzyloxy-3-fluor)phenyl]-pyrimidin
- Bsp. 46: 2-Brom-5-[(4-benzyloxy-2-fluor)phenyl]-pyrimidin
- Bsp. 47: 2-Brom-5-[(4-benzyloxy)phenyl]pyridin
- Bsp. 48: 2-Brom-5-[(4-benzyloxy-2,3-difluor)phenyl]-pyridin
- Bsp. 49: 2-Brom-5-[(4-benzyloxy-3-fluor)phenyl]-pyridin
- Bsp. 50: 2-Brom-5-[(4-benzyloxy-2-fluor)phenyl]-pyridin
- Bsp. 51: 2-Chlor-5-[(4-benzyloxy)phenyl]pyridin
- Bsp. 52: 2-Chlor-5-[(4-benzyloxy-2,3-difluor)phenyl]-pyridin
- Bsp. 53: 2-Chlor-5-[(4-benzyloxy-3-fluor)phenyl]-pyridin
- Bsp. 54: 2-Chlor-5-[(4-benzyloxy-2-fluor)phenyl]-pyridin

### Beispiel 55

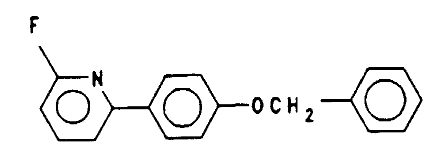

Eine Lösung von 17.6 g 2-Brom-6-fluor-pyridin (Herstellung wie in DE-A 4 040 575 beschrieben), 22.8 g 4-Benzyloxy-phenylboronsäure und 21.2 g Natriumcarbonat in 300 ml Toluol, 150 ml Ethanol und 100 ml Wasser wird unter Zusatz von Tetrakis(triphenylphosphin)palladium (O) für 2 h zum Rückfluß erhitzt. Die organische Phase wird abgetrennt und im Vakuum zur Trockne gebracht: 31 g Rohprodukt; nach Umkristallisation aus 220 ml Acetonitril werden 20.8 g Produkt erhalten.

Analog werden die Beispiel 56-58 erhalten:
- Bsp. 56: 6-Fluor-2-[(4-benzyloxy-2,3-difluor)phenyl]-pyridin
- Bsp. 57: 6-Fluor-2-[(4-benzyloxy-3-fluor)phenyl]-pyridin
- Bsp. 58: 6-Fluor-2-[(4-benzyloxy-2-fluor)phenyl]-pyridin

### Beispiel 59

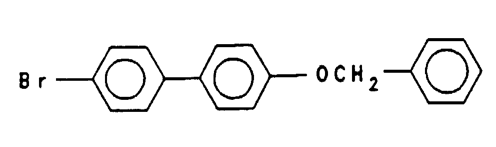

Eine Lösung von 37.2 g 4-Bromphenyl-trifluormethansulfonsäureester, 34.2 g 4-Benzyloxy-phenylboronsäure, 1.8 g Tetrakis(triphenylphosphin)palladium (O) und 32 g Natriumcarbonat in 450 ml Toluol, 225 ml Ethanol und 150 ml Wasser wird 12 h zum Rückfluß erhitzt. Nach Aufarbeitung wie in Beispiel 1 und Umkristallisation aus Acetonitril werden 34.2 g farblose Kristalle v. Schmp. 157-158°C erhalten.

Analog werden die Beispiele 60 - 127 erhalten:
- Bsp. 60: 4-Brom-4'-benzyloxy-2',3'-difluor-biphenyl
- Bsp. 61: 4-Brom-4'-benzyloxy-2'-fluor-biphenyl
- Bsp. 62: 4-Brom-4'-benzyloxy-3'-fluor-biphenyl
- Bsp. 63: 4-Brom-3-fluor-4'-benzyloxy-biphenyl
- Bsp. 64: 4-Brom-3-fluor-4'-benzyloxy-2',3'-difluor-biphenyl
- Bsp. 65: 4-Brom-3-fluor-4'-benzyloxy-2'-fluor-biphenyl
- Bsp. 66: 4-Brom-3-fluor-4'-benzyloxy-3'-fluor-biphenyl
- Bsp. 67: 4-Brom-2-fluor-4'-benzyloxy-biphenyl
- Bsp. 68: 4-Brom-2-fluor-4'-benzyloxy-2',3'-difluor-biphenyl
- Bsp. 69: 4-Brom-2-fluor-4'-benzyloxy-2'-fluor-biphenyl
- Bsp. 70: 4-Brom-2-fluor-4'-benzyloxy-3'-fluor-biphenyl
- Bsp. 71: 4-Brom-2,3-difluor-4'-benzyloxy-biphenyl
- Bsp. 72: 4-Brom-2,3-difluor-4'-benzyloxy-2',3'-difluor-biphenyl
- Bsp. 73: 4-Brom-2,3-difluor-4'-benzyloxy-2'-fluor-biphenyl
- Bsp. 74: 4-Brom-2,3-difluor-4'-benzyloxy-3'-fluor-biphenyl
- Bsp. 75: 4-Brom-4"-benzyloxy-p-terphenyl
- Bsp. 76: 4-Brom-4"-benzyloxy-2",3"-difluor-p-terphenyl
- Bsp. 77: 4-Brom-4"-benzyloxy-2"-fluor-p-terphenyl
- Bsp. 78: 4-Brom-4"-benzyloxy-3"-fluor-p-terphenyl
- Bsp. 79: 4-Brom-4"-benzyloxy-2',3'-difluor-p-terphenyl
- Bsp. 80: 4-Brom-4"-benzyloxy-2'-fluor-p-terphenyl
- Bsp. 81: 4-Brom-4"-benzyloxy-3'-fluor-p-terphenyl
- Bsp. 82: 4-Brom-3-fluor-4"-benzyloxy-p-terphenyl
- Bsp. 83: 4-Brom-3-fluor-4"-benzyloxy-2",3"-difluor-p-terphenyl
- Bsp. 84: 4-Brom-3-fluor-4"-benzyloxy-2',3'-difluor-p-terphenyl
- Bsp. 85: 4-Brom-3-fluor-4"-benzyloxy-2',2",3',3"-tetrafluor-p-terphenyl
- Bsp. 86: 4-Brom-3-fluor-4"-benzyloxy-2',2",3'-trifluor-p-terphenyl
- Bsp. 87: 4-Brom-3-fluor-4"-benzyloxy-2',2",3"-trifluor-p-terphenyl
- Bsp. 88: 4-Brom-3-fluor-4"-benzyloxy-2',2"-difluor-p-terphenyl
- Bsp. 89: 4-Brom-3-fluor-4"-benzyloxy-2',3"-difluor-p-terphenyl
- Bsp. 90: 4-Brom-3-fluor-4"-benzyloxy-3',3"-difluor-p-terphenyl
- Bsp. 91: 4-Brom-3-fluor-4"-benzyloxy-3',2"-difluor-p-terphenyl
- Bsp. 92: 4-Brom-3-fluor-4"-benzyloxy-2'-fluor-p-terphenyl
- Bsp. 93: 4-Brom-3-fluor-4"-benzyloxy-3'-fluor-p-terphenyl
- Bsp. 94: 4-Brom-3-fluor-4"-benzyloxy-2"-fluor-p-terphenyl
- Bsp. 95: 4-Brom-3-fluor-4"-benzyloxy-3"-fluor-p-terphenyl
- Bsp. 96: 4-Brom-2-fluor-4"-benzyloxy-p-terphenyl
- Bsp. 97: 4-Brom-2-fluor-4"-benzyloxy-2",3"-difluor-p-terphenyl
- Bsp. 98: 4-Brom-2-fluor-4"-benzyloxy-2',3'-difluor-p-terphenyl
- Bsp. 99: 4-Brom-2-fluor-4"-benzyloxy-2',2",3',3"-tetrafluor-p-terphenyl
- Bsp.100: 4-Brom-2-fluor-4"-benzyloxy-2',2",3'-trifluor-p-terphenyl
- Bsp.101: 4-Brom-2-fluor-4"-benzyloxy-2',2",3"-trifluor-p-terphenyl
- Bsp.102: 4-Brom-2-fluor-4"-benzyloxy-2',2"-difluor-p-terphenyl
- Bsp.103: 4-Brom-2-fluor-4"-benzyloxy-2',3"-difluor-p-terphenyl
- Bsp.104: 4-Brom-2-fluor-4"-benzyloxy-3',3"-difluor-p-terphenyl
- Bsp.105: 4-Brom-2-fluor-4"-benzyloxy-3',2"-difluor-p-terphenyl
- Bsp.106: 4-Brom-2-fluor-4"-benzyloxy-2'-fluor-p-terphenyl
- Bsp.107: 4-Brom-2-fluor-4"-benzyloxy-3'-fluor-p-terphenyl
- Bsp.108: 4-Brom-2-fluor-4"-benzyloxy-2"-fluor-p-terphenyl
- Bsp.109: 4-Brom-2-fluor-4"-benzyloxy-3"-fluor-p-terphenyl
- Bsp.110: 4-Brom-2,3-difluor-4"-benzyloxy-p-terphenyl
- Bsp.111: 4-Brom-2,3-difluor-4"-benzyloxy-2",3"-difluor-p-terphenyl
- Bsp.112: 4-Brom-2,3-difluor-4"-benzyloxy-2',3'-difluor-p-terphenyl
- Bsp.113: 4-Brom-2,3-difluor-4"-benzyloxy-2',2",3',3"-tetrafluor-p-terphenyl
- Bsp.114: 4-Brom-2,3-difluor-4"-benzyloxy-2',2",3'-trifluor-p-terphenyl
- Bsp. 115: 4-Brom-2,3-difluor-4"-benzyloxy-2',2",3"-trifluor-p-terphenyl
- Bsp.116: 4-Brom-2,3-difluor-4"-benzyloxy-2',2"-difluor-p-terphenyl
- Bsp.117: 4-Brom-2,3-difluor-4"-benzyloxy-2',3"-difluor-p-terphenyl
- Bsp.118: 4-Brom-2,3-difluor-4"-benzyloxy-3',3"-difluor-p-terphenyl
- Bsp.119: 4-Brom-2,3-difluor-4"-benzyloxy-3',2"-difluor-p-terphenyl
- Bsp.120: 4-Brom-2,3-difluor-4"-benzyloxy-2'-fluor-p-terphenyl
- Bsp.121: 4-Brom-2,3-difluor-4"-benzyloxy-3'-fluor-p-terphenyl
- Bsp.122: 4-Brom-2,3-difluor-4"-benzyloxy-2"-fluor-p-terphenyl
- Bsp.123: 4-Brom-2,3-difluor-4"-benzyloxy-3"-fluor-p-terphenyl
- Bsp.124: 6-Brom-2-(4-benzyloxy)phenyl-naphthalin
- Bsp.125: 6-Brom-2-(4-benzyloxy-2,3-difluor)phenyl-naphthalin
- Bsp.126: 6-Brom-2-(4-benzyloxy-2-fluor)phenyl-naphthalin
- Bsp.127: 6-Brom-2-(4-benzyloxy-3-fluor)phenyl-naphthalin

### Beispiel 128

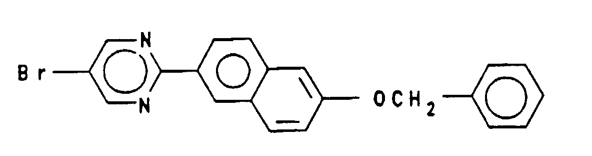

wird analog Beispiel 1 erhalten durch Kupplung zwischen 2,5-Dibrompyrimidin und 6-Benzyloxy-naphthalin-2-boronsäure.

Analog werden die Beispiele 129-131 erhalten:
- Bsp.129: 5-Brom-2-[6-benzyloxy-naphthalin-2-yl]-pyridin
- Bsp.130: 2-Brom-5-[6-benzyloxy-naphthalin-2-yl]-pyrimidin
- Bsp.131: 2-Brom-5-[6-benzyloxy-naphthalin-2-yl]-pyridin

### Beispiel 132

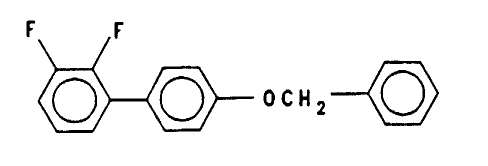

wird analog Beispiel 59 erhalten durch Kupplung zwischen 2,3-Difluorphenylboronsäure und 4-Benzyloxy-brombenzol; fbls.Kristalle v. Schmp. 82-83°C.

Analog werden die Beispiele 133-169 erhalten:
- Bsp. 133: 3-Fluor-4'-benzyloxy-biphenyl
- Bsp. 134: 3-Fluor-4'-benzyloxy-2',3'-difluor-biphenyl
- Bsp. 135: 3-Fluor-4'-benzyloxy-2'-fluor-biphenyl
- Bsp. 136: 3-Fluor-4'-benzyloxy-3'-fluor-biphenyl
- Bsp. 137: 2,3-Difluor-4'-benzyloxy-biphenyl
- Bsp. 138: 2,3-Difluor-4'-benzyloxy-2',3'-difluor-biphenyl
- Bsp. 139: 2,3-Difluor-4'-benzyloxy-2'-fluor-biphenyl
- Bsp. 140: 2,3-Difluor-4'-benzyloxy-3'-fluor-biphenyl
- Bsp. 141: entfällt
- Bsp. 142: 3-Fluor-4"-benzyloxy-p-terphenyl
- Bsp. 143: 3-Fluor-4"-benzyloxy-2",3"-difluor-p-terphenyl
- Bsp. 144: 3-Fluor-4"-benzyloxy-2',3'-difluor-p-terphenyl
- Bsp. 145: 3-Fluor-4"-benzyloxy-2',2",3',3"-tetrafluor-p-terphenyl
- Bsp. 146: 3-Fluor-4"-benzyloxy-2',2",3'-trifluor-p-terphenyl
- Bsp. 147: 3-Fluor-4"-benzyloxy-2',2",3"-trifluor-p-terphenyl
- Bsp. 148: 3-Fluor-4"-benzyloxy-2',2"-difluor-p-terphenyl
- Bsp. 149: 3-Fluor-4"-benzyloxy-2',3"-difluor-p-terphenyl
- Bsp. 150: 3-Fluor-4"-benzyloxy-3',3"-difluor-p-terphenyl
- Bsp. 151: 3-Fluor-4"-benzyloxy-3',2"-difluor-p-terphenyl
- Bsp. 152: 3-Fluor-4"-benzyloxy-2'-fluor-p-terphenyl
- Bsp. 153: 3-Fluor-4"-benzyloxy-3'-fluor-p-terphenyl
- Bsp. 154: 3-Fluor-4"-benzyloxy-2"-fluor-p-terphenyl
- Bsp. 155: 3-Fluor-4"-benzyloxy-3"-fluor-p-terphenyl
- Bsp. 156: 2,3-Difluor-4"-benzyloxy-p-terphenyl
- Bsp. 157: 2,3-Difluor-4"-benzyloxy-2",3"-difluor-p-terphenyl
- Bsp. 158: 2,3-Difluor-4"-benzyloxy-2',3'-difluor-p-terphenyl
- Bsp. 159: 2,3-Difluor-4"-benzyloxy-2',2",3',3"-tetrafluor-p-terphenyl
- Bsp. 160: 2,3-Difluor-4"-benzyloxy-2',2",3'-trifluor-p-terphenyl
- Bsp. 161: 2,3-Difluor-4"-benzyloxy-2',2",3"-trifluor-p-terphenyl
- Bsp. 162: 2,3-Difluor-4"-benzyloxy-2',2"-difluor-p-terphenyl
- Bsp. 163: 2,3-Difluor-4"-benzyloxy-2',3"-difluor-p-terphenyl
- Bsp. 164: 2,3-Difluor-4"-benzyloxy-3',3"-difluor-p-terphenyl
- Bsp. 165: 2,3-Difluor-4"-benzyloxy-3',2"-difluor-p-terphenyl
- Bsp. 166: 2,3-Difluor-4"-benzyloxy-2'-fluor-p-terphenyl
- Bsp. 167: 2,3-Difluor-4"-benzyloxy-3'-fluor-p-terphenyl
- Bsp. 168: 2,3-Difluor-4"-benzyloxy-2"-fluor-p-terphenyl
- Bsp. 169: 2,3-Difluor-4"-benzyloxy-3"-fluor-p-terphenyl

## Patentansprüche (Patentansprüche für folgende(n) Vertragsstaat(en): DE, FR, GB)

1. Bifunktionelle Verbindungen der Formel (I), 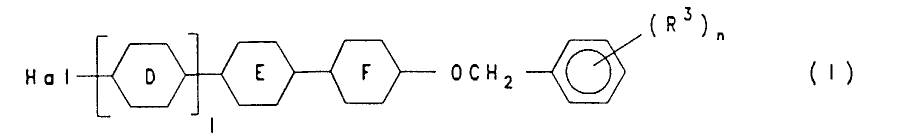 worin bedeuten: 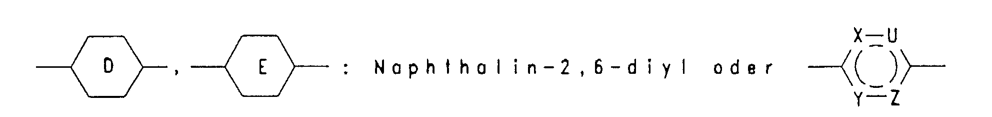 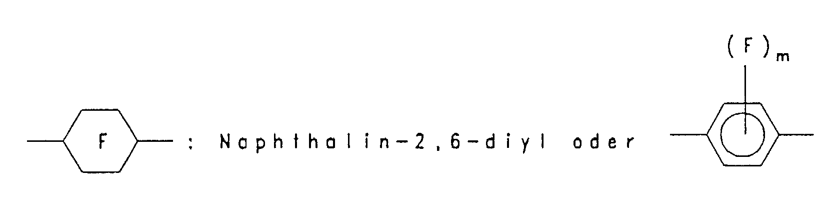
U, X, Y, Z : -CH =, -CF = und -N =, mit der Maßgabe, daß -CF= bzw. -N = jeweils nur höchstens zweimal pro Sechsring vertreten sein dürfen und daß in einem Sechsring -CF = und -N = nicht zugleich zweimal vertreten sein dürfen;
Hal: Cl, Br oder I; oder H, wenn X oder/und Y=CF=sind
R³: CH₃ oder OCH₃
I: 0 oder 1
m, n: 0, 1, 2 oder 3,
wobei die folgende Verbindung ausgenommen ist:

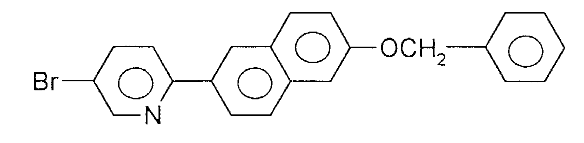

2. Bifunktionelle Verbindungen nach Anspruch 1, dadurch gekennzeichnet, daß die Symbole in der allgemeinen Formel I
I = 0
U,X,Y,Z: mindestens einmal -N = und höchstens einmal -CF=, ansonsten -CH =
m: 0, 1, 2 oder 3
n: 0,1 oder 2
R³: CH₃ oder OCH₃
Hal: Br oder I
bedeuten.

3. Bifunktionelle Verbindungen nach Anspruch 1 oder 2, dadurch gekennzeichnet, daß die Symbole in der allgemeinen Formel I
I = 0
U,X,Y,Z: ein- oder zweimal -N =, ansonsten -CH =
m: 0, 1, 2 oder 3
n: 0
Hal: Br oder I
bedeuten.

4. Verfahren zur Darstellung der bifunktionellen Verbindungen nach einem oder mehreren der Ansprüche 1 bis 3, dadurch gekennzeichnet, daß eine Arylboronsäure der allgemeinen Formel XXI, 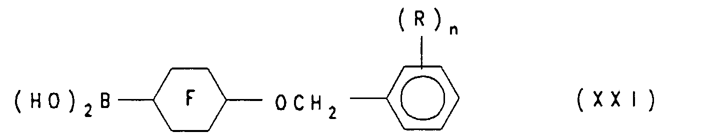 in der die Substituenten und Indizes die folgende Bedeutung aufweisen: 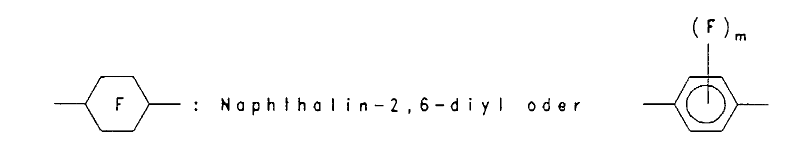
R: CH₃, OCH₃
m: 0, 1, 2 oder 3
n: 0, 1 oder 2
mit einer Halogenverbindung der allgemeinen Formel XXII 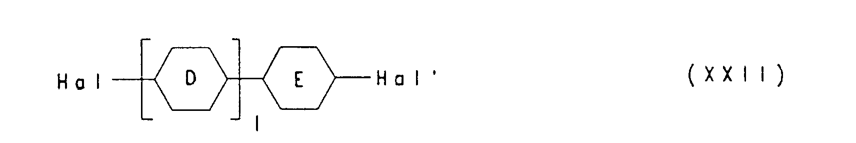 in der
Hal: Cl, Br, I und
Hal': Cl, Br, I und Perfluoralkansulfonat
bedeutet
unter Katalyse durch Palladium oder eine Palladiumverbindung zu einer Verbindung der allgemeinen Formel I gekuppelt wird.

5. Verwendung von bifunktionellen Verbindungen nach einem oder mehreren der Ansprüche 1 bis 3 als Zwischenprodukte zur Herstellung von Komponenten von Flüssigkristallmischungen.

6. Verwendung nach Anspruch 5, dadurch gekennzeichnet, daß die Flüssigkristallmischung ferroelektrisch ist.

7. Verwendung nach Anspruch 5, dadurch gekennzeichnet, daß die Flüssigkristallmischung nematisch ist.

8. Verwendung von bifunktionellen Verbindungen nach einem oder mehreren der Ansprüche 1 bis 3 als Komponenten von Flüssigkristallmischungen.

9. Zwischenprodukt, zur Herstellung von Komponenten für Flüssigkristallmischungen, der Formel (VI) 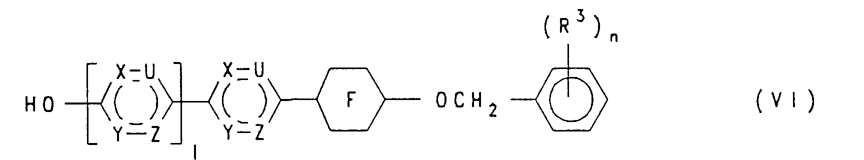 wobei die Symbole die in Formel (I) in Anspruch 1 angegebenen Bedeutungen haben und für den die OH-Funktion tragenden Ring U und/oder Z -N =, X und/oder Y jedoch nicht -N = bedeuten sollen.

10. Zwischenprodukte, zur Herstellung von Komponenten für Flüssigkristallmischungen, der Formel (VII) 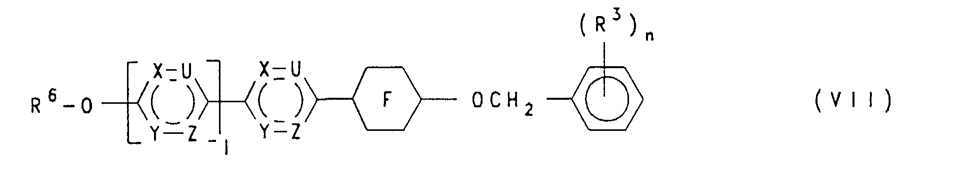 worin 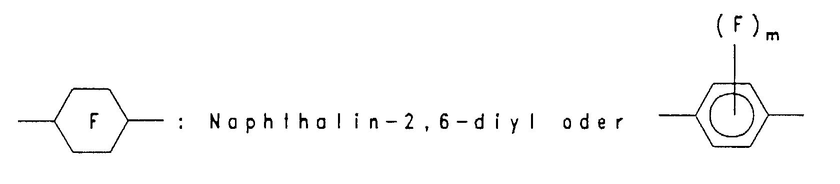
X, Y, U, Z: -CH = , -CF = und -N =, mit der Maßgabe, daß -CF= bzw. -N = jeweils nur höchstens zweimal pro Sechsring vertreten sein dürfen und daß in einem Sechsring -CF = und -N = nicht zugleich zweimal vertreten sein dürfen und daß in dem an R⁶-O- gebundenen Ring mindestens ein Stickstoffatom vorhanden sein muß und daß in diesem Ring X und/oder Y nicht N sein dürfen;
R³: CH₃ oder OCH₃;
R⁶: Alkyl mit 1 bis 18 C-Atomen, worin auch eine oder mehrere nicht benachbarte -CH₂-Gruppen durch -O-, -CH = CH- oder -Si(CH₃)₂)- ersetzt sein können,
I: 0 oder 1;
m, n: 0, 1, 2 oder 3 bedeuten.

11. Zwischenprodukte, zur Herstellung von Komponenten für Flüssigkristallmischungen, der Formel (VIII) 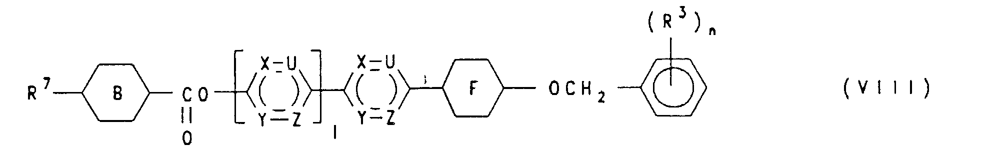 worin 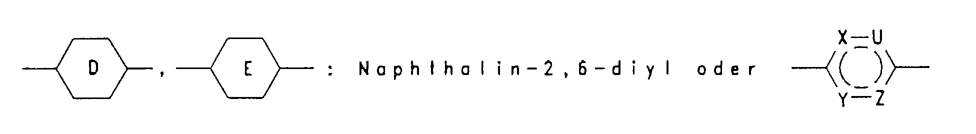 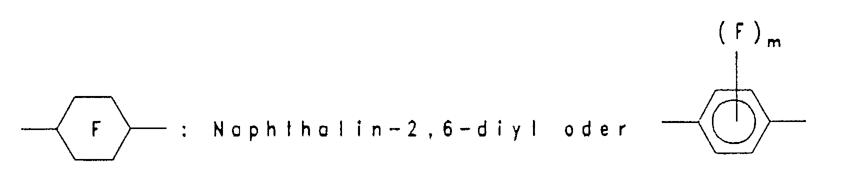 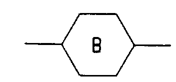 1,4-Phenylen, gegebenenfalls 1 oder 2 mal durch F substituiert, oder 1,4-Cyclohexylen oder 1,3-Dioxan-2,5-diyl.
U, X, Y, Z : -CH =, -CF= und -N =, mit der Maßgabe, daß -CF= bzw. - N = jeweils nur höchstens zweimal pro Sechsring vertreten sein dürfen und in einem Sechsring -CF = und -N = nicht zugleich zweimal vertreten sein dürfen;
R³: CH₃ oder OCH₃
R⁷: Alkyl mit 1 bis 18 C-Atomen, worin auch eine oder mehrere nicht benachbarte -CH₂-Gruppen durch -O-, -CH = CH-oder - Si(CH₃)₂-ersetzt sein können;
I: 0 oder 1;
m, n: 0, 1, 2 oder 3 bedeuten.

12. Zwischenprodukte, zur Herstellung von Komponenten für Flüssigkristallmischungen, der Formel (XIII), 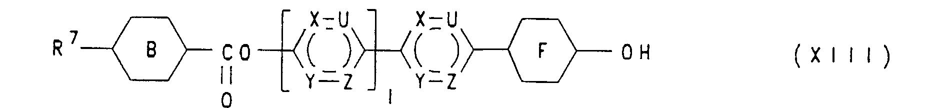 worin die Symbole die in Formel (VIII) in Anspruch 11 angegebenen Bedeutungen haben.

## Patentansprüche (Patentansprüche für folgende(n) Vertragsstaat(en): CH, LI)

1. Bifunktionelle Verbindungen der Formel (I), 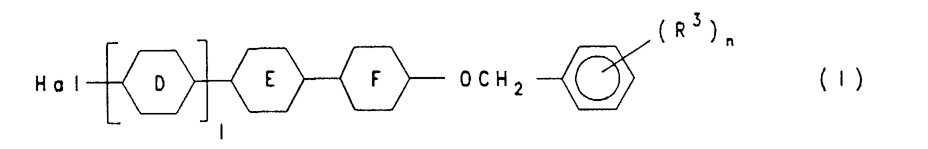 worin bedeuten: 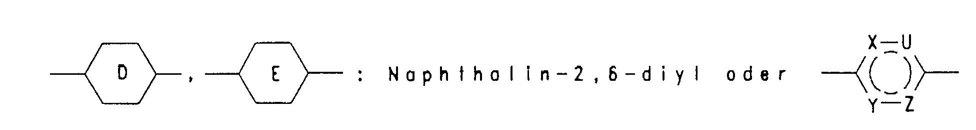 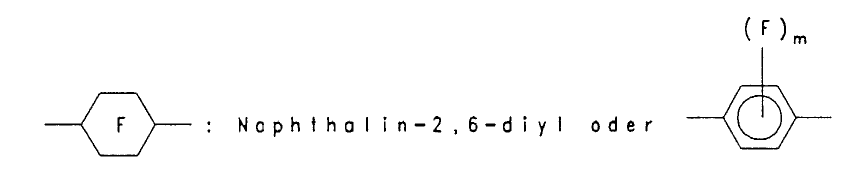
U, X, Y, Z : -CH =, -CF= und -N =, mit der Maßgabe, daß -CF= bzw. -N = jeweils nur höchstens zweimal pro Sechsring vertreten sein dürfen und daß in einem Sechsring -CF = und -N = nicht zugleich zweimal vertreten sein dürfen;
Hal: Cl, Br oder I; oder H, wenn X oder/und Y=CF=sind
R³: CH₃ oder OCH₃
I: 0 oder 1
m, n: 0, 1, 2 oder 3,

2. Bifunktionelle Verbindungen nach Anspruch 1, dadurch gekennzeichnet, daß die Symbole in der allgemeinen Formel I
I = 0
U,X,Y,Z: mindestens einmal -N = und höchstens einmal -CF =, ansonsten -CH =
m: 0, 1, 2 oder 3
n: 0,1 oder 2
R³: CH₃ oder OCH₃
Hal: Br oder I
bedeuten.

3. Bifunktionelle Verbindungen nach Anspruch 1 oder 2, dadurch gekennzeichnet, daß die Symbole in der allgemeinen Formel I
I = 0
U,X,Y,Z: ein- oder zweimal -N =, ansonsten -CH =
m: 0, 1, 2 oder 3
n: 0
Hal: Br oder I
bedeuten.

4. Verfahren zur Darstellung der bifunktionellen Verbindungen nach einem oder mehreren der Ansprüche 1 bis 3, dadurch gekennzeichnet, daß eine Arylboronsäure der allgemeinen Formel XXI, 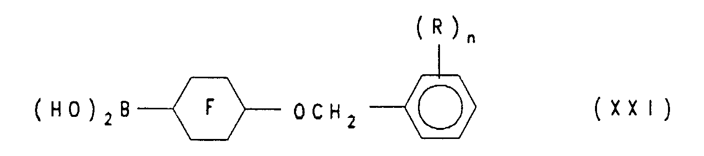 in der die Substituenten und Indizes die folgende Bedeutung aufweisen: 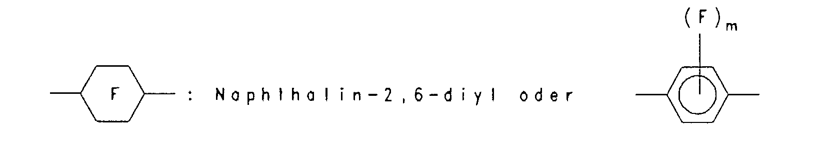
R: CH₃, OCH₃
m: 0, 1, 2 oder 3
n: 0, 1 oder 2
mit einer Halogenverbindung der allgemeinen Formel XXII 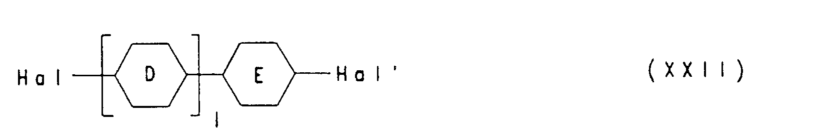 in der
Hal: Cl, Br, I und
Hal': Cl, Br, I und Perfluoralkansulfonat
bedeutet
unter Katalyse durch Palladium oder eine Palladiumverbindung zu einer Verbindung der allgemeinen Formel I gekuppelt wird.

5. Verwendung von bifunktionellen Verbindungen nach einem oder mehreren der Ansprüche 1 bis 3 als Zwischenprodukte zur Herstellung von Komponenten von Flüssigkristallmischungen.

6. Verwendung nach Anspruch 5, dadurch gekennzeichnet, daß die Flüssigkristallmischung ferroelektrisch ist.

7. Verwendung nach Anspruch 5, dadurch gekennzeichnet, daß die Flüssigkristallmischung nematisch ist.

8. Verwendung von bifunktionellen Verbindungen nach einem oder mehreren der Ansprüche 1 bis 3 als Komponenten von Flüssigkristallmischungen.

9. Zwischenprodukt, zur Herstellung von Komponenten für Flüssigkristallmischungen, der Formel (VI) 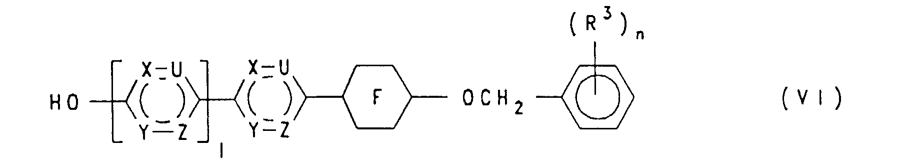 wobei die Symbole die in Formel (I) in Anspruch 1 angegebenen Bedeutungen haben und für den die OH-Funktion tragenden Ring U und/oder Z -N =, X und/oder Y jedoch nicht -N = bedeuten sollen.

10. Zwischenprodukte, zur Herstellung von Komponenten für Flüssigkristallmischungen, der Formel (VII) 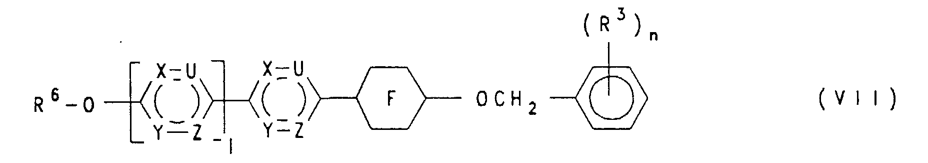 worin 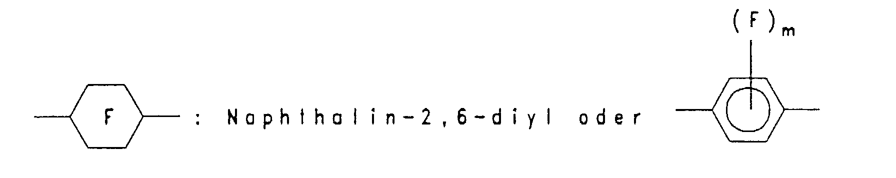
X, Y, U, Z: -CH = , -CF = und -N =, mit der Maßgabe, daß -CF = bzw. -N = jeweils nur höchstens zweimal pro Sechsring vertreten sein dürfen und daß in einem Sechsring -CF = und -N = nicht zugleich zweimal vertreten sein dürfen und daß in dem an R⁶-O- gebundenen Ring mindestens ein Stickstoffatom vorhanden sein muß und daß in diesem Ring X und/oder Y nicht N sein dürfen;
R³: CH₃ oder OCH₃;
R⁶: Alkyl mit 1 bis 18 C-Atomen, worin auch eine oder mehrere nicht benachbarte -CH₂-Gruppen durch -O-, -CH=CH- oder -Si(CH₃)₂)- ersetzt sein können,
I: 0 oder 1;
m, n: 0, 1, 2 oder 3 bedeuten.

11. Zwischenprodukte, zur Herstellung von Komponenten für Flüssigkristallmischungen, der Formel (VIII) 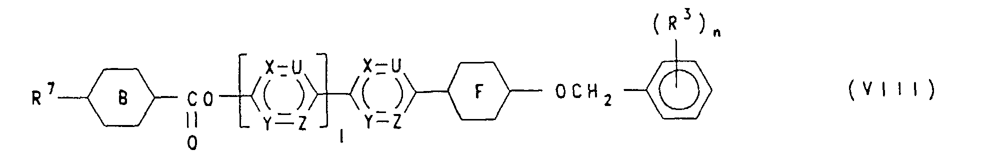 worin 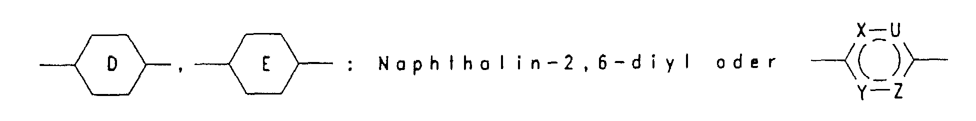 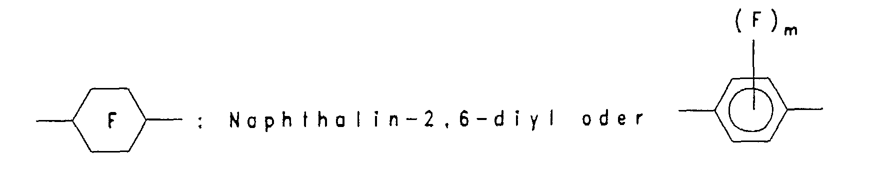 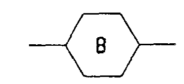 1,4-Phenylen, gegebenenfalls 1 oder 2 mal durch F substituiert, oder 1,4-Cyclohexylen oder 1,3-Dioxan-2,5-diyl;
U, X, Y, Z : -CH =, -CF= und -N =, mit der Maßgabe, daß -CF = bzw. - N = jeweils nur höchstens zweimal pro Sechsring vertreten sein dürfen und in einem Sechsring -CF = und -N = nicht zugleich zweimal vertreten sein dürfen;
R³: CH₃ oder OCH₃
R⁷: Alkyl mit 1 bis 18 C-Atomen, worin auch eine oder mehrere nicht benachbarte -CH₂-Gruppen durch -O-, -CH = CH-oder -Si(CH₃)₂-ersetzt sein können;
I: 0 oder 1;
m, n: 0, 1, 2 oder 3 bedeuten.

12. Zwischenprodukte, zur Herstellung von Komponenten für Flüssigkristallmischungen, der Formel (XIII), 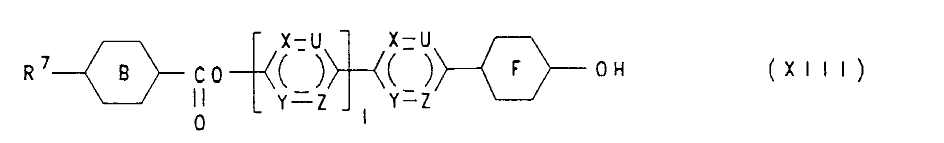 worin die Symbole die in Formel (VIII) in Anspruch 11 angegebenen Bedeutungen haben.

## Claims (Claims for the following Contracting State(s): DE, FR, GB)

1. A bifunctional compound of the formula (I) 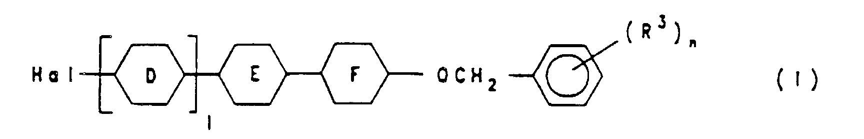 in which: 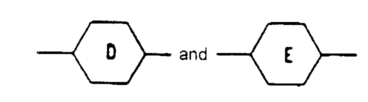 are naphthalene-2,6-diyl or 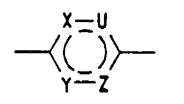 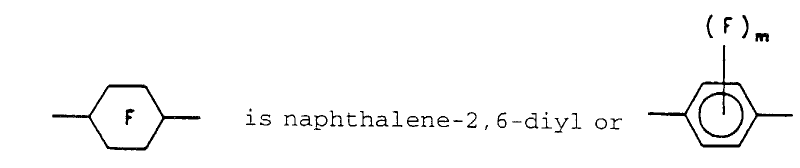
U, X, Y and Z are -CH=, -CF= and -N=, with the proviso that -CF= and -N= may each only be represented at most twice per six-membered ring, and that -CF= and -N= must not simultaneously be represented twice in one six-membered ring;
Hal is Cl, Br or I; or H if X and/or Y are -CF=
R³ is CH₃ or OCH₃
l is 0 or 1 and
m and n are 0, 1, 2 or 3,
with the exception of the following compound:

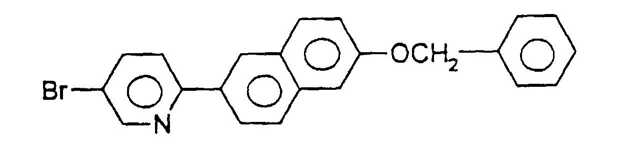

2. A bifunctional compound as claimed in claim 1, wherein the symbols in the formula I have the following meanings:
l = 0
U, X, Y and Z: at least once -N= and at most once -CF=, otherwise -CH=
m: 0, 1, 2 or 3
n: 0, 1 or 2
R³: CH₃ or OCH₃
Hal: Br or I.

3. A bifunctional compound as claimed in claim 1 or 2, wherein the symbols in the formula I have the following meanings:
l = 0
U, X, Y and Z: once or twice -N=, otherwise -CH=
m: 0, 1, 2 or 3
n: 0
Hal: Br or I.

4. A process for the preparation of a bifunctional compound as claimed in one or more of claims 1 to 3, which comprises coupling an arylboronic acid of the formula XXI 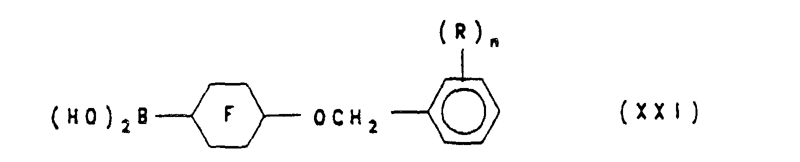 in which the substituents and indices have the following meanings: 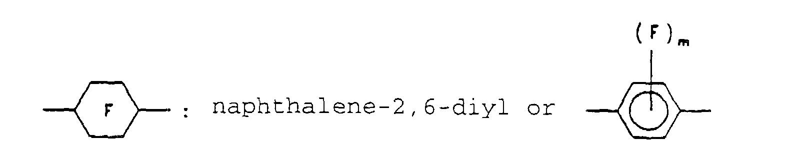
R: CH₃ or OCH₃
m: 0, 1, 2 or 3
n: 0, 1 or 2
with a halogen compound of the formula XXII 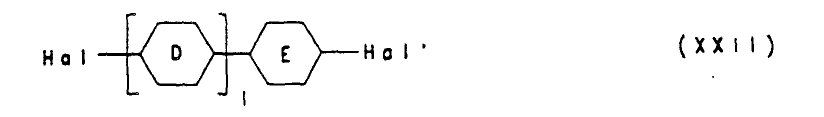 in which
Hal is Cl, Br, I, and
Hal' is Cl, Br, I and perfluoroalkanesulphonate,
with catalysis by palladium or a palladium compound, to give a compound of the formula I.

5. The use of a bifunctional compound as claimed in one or more of claims 1 to 3 as an intermediate for the preparation of components of liquid-crystal mixtures.

6. The use as claimed in claim 5, wherein the liquid-crystal mixture is ferroelectric.

7. The use as claimed in claim 5, wherein the liquid-crystal mixture is nematic.

8. The use of a bifunctional compound as claimed in one or more of claims 1 to 3 as a component of liquid-crystal mixtures.

9. An intermediate, for the preparation of components for liquid-crystal mixtures, of the formula (VI) 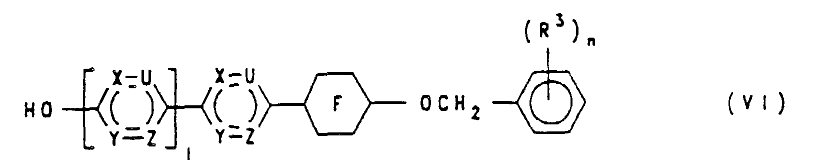 where the symbols are as defined in the formula (I) in claim 1 and, for the ring carrying the OH function, U and/or Z are -N=, but X and/or Y are not -N=.

10. An intermediate, for the preparation of components for liquid-crystal mixtures, of the formula (VII) 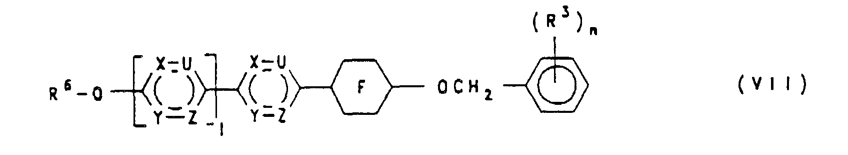 in which 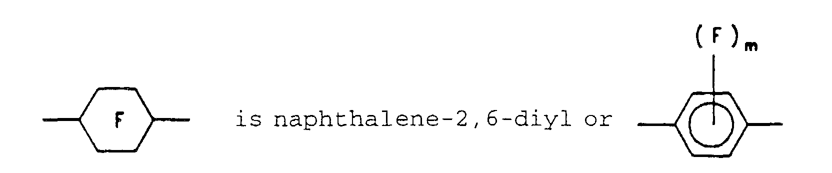
X, Y, U and Z are -CH=, -CF= and -N=, with the proviso that -CF= and -N= may each only be represented at most twice per six-membered ring and that -CF= and -N= must not simultaneously be represented twice in one six-membered ring, and that at least one nitrogen atom must be present in the ring bonded to R⁶-O-, and that X and/or Y must not be N in this ring;
R³ is CH₃ or OCH₃;
R⁶ is alkyl having 1 to 18 carbon atoms, in which, in addition, one or more non-adjacent -CH₂-groups may be replaced by -O-, -CH=CH- or -Si(CH₃)₂-,
l is 0 or 1; and
m and n are 0, 1, 2 or 3.

11. An intermediate, for the preparation of components for liquid-crystal mixtures, of the formula (VIII) 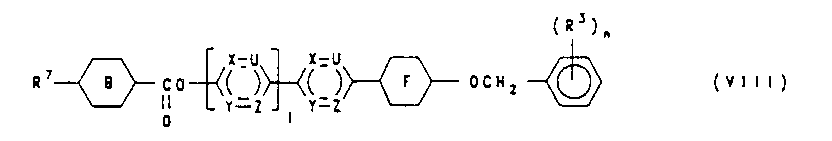 in which 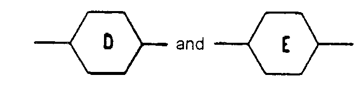 are naphthalene-2,6-diyl or 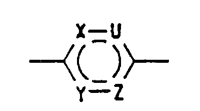 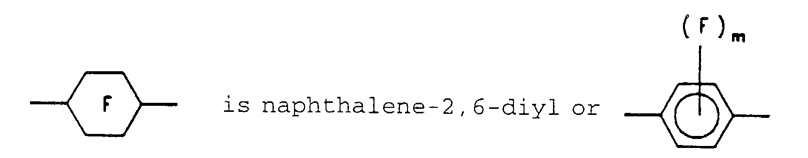 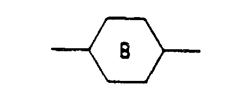 is 1,4-phenylene, unsubstituted or monosubstituted or disubstituted by F, or 1,4-cyclohexylene or 1,3-dioxane-2,5-diyl;
U, X, Y and Z are -CH=, -CF= and -N=, with the proviso that -CF= and -N= may each only be represented twice per six-membered ring, and -CF= and -N= must not simultaneously be represented twice in one six-membered ring;
R³ is CH₃ or OCH₃
R⁷ is alkyl having 1 to 18 carbon atoms, in which, in addition, one or more non-adjacent -CH₂-groups may be replaced by -O-, -CH=CH- or -Si(CH₃)₂;
l is 0 or 1; and
m and n are 0, 1, 2 or 3.

12. An intermediate, for the preparation of components for liquid-crystal mixtures, of the formula (XIII) 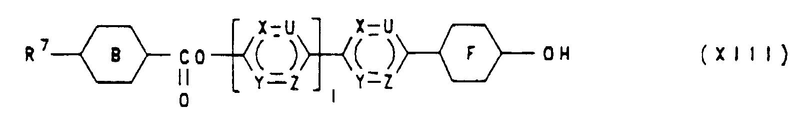 in which the symbols are as defined in the formula (VIII) in claim 11.

## Claims (Claims for the following Contracting State(s): CH, LI)

1. A bifunctional compound of the formula (I) 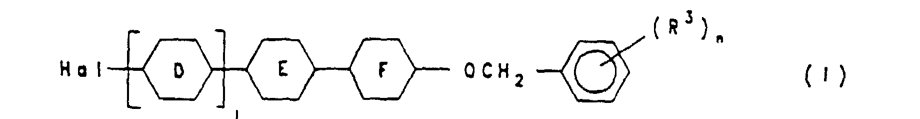 in which: 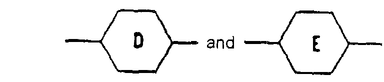 are naphthalene-2,6-diyl or 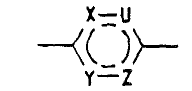 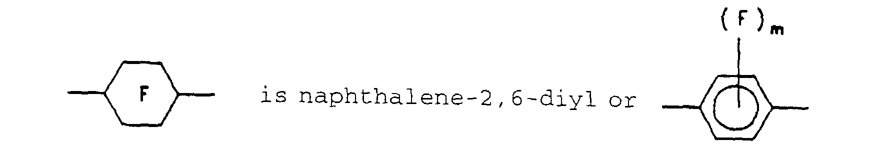
U, X, Y and Z are -CH=, -CF= and -N=, with the proviso that -CF= and -N= may each only be represented at most twice per six-membered ring, and that -CF= and -N= must not simultaneously be represented twice in one six-membered ring;
Hal is Cl, Br or I; or H if X and/or Y are -CF=
R³ is CH₃ or OCH₃
I is 0 or 1 and
m and n are 0, 1, 2 or 3.

2. A bifunctional compound as claimed in claim 1, wherein the symbols in the formula I have the following meanings:
l = 0
U, X, Y and Z: at least once -N= and at most once -CF=, otherwise -CH=
m: 0, 1, 2 or 3
n: 0, 1 or 2
R³: CH₃ or OCH₃
Hal: Br or I.

3. A bifunctional compound as claimed in claim 1 or 2, wherein the symbols in the formula I have the following meanings:
l = 0
U, X, Y and Z: once or twice -N=, otherwise -CH=
m: 0, 1, 2 or 3
n: 0
Hal: Br or I.

4. A process for the preparation of a bifunctional compound as claimed in one or more of claims 1 to 3, which comprises coupling an arylboronic acid of the formula XXI 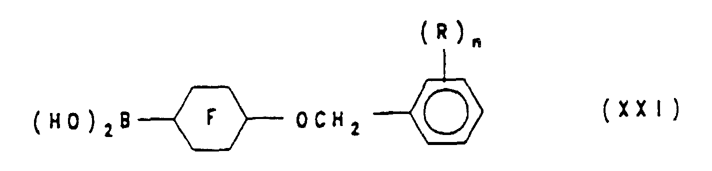 in which the substituents and indices have the following meanings: 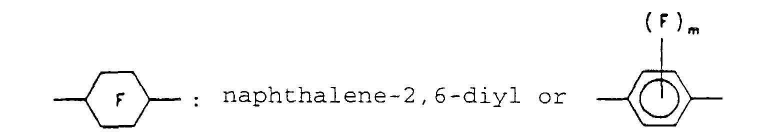
R: CH₃ or OCH₃
m: 0, 1, 2 or 3
n: 0, 1 or 2
with a halogen compound of the formula XXII 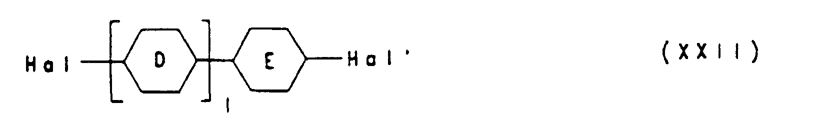 in which
Hal is Cl, Br, I, and
Hal' is Cl, Br, I and perfluoroalkanesulphonate,
with catalysis by palladium or a palladium compound, to give a compound of the formula I.

5. The use of a bifunctional compound as claimed in one or more of claims 1 to 3 as an intermediate for the preparation of components of liquid-crystal mixtures.

6. The use as claimed in claim 5, wherein the liquid-crystal mixture is ferroelectric.

7. The use as claimed in claim 5, wherein the liquid-crystal mixture is nematic.

8. The use of a bifunctional compound as claimed in one or more of claims 1 to 3 as a component of liquid-crystal mixtures.

9. An intermediate, for the preparation of components for liquid-crystal mixtures, of the formula (VI) 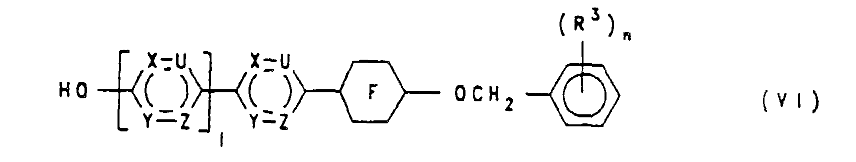 where the symbols are as defined in the formula (I) in claim 1 and, for the ring carrying the OH function, U and/or Z are -N=, but X and/or Y are not -N=.

10. An intermediate, for the preparation of components for liquid-crystal mixtures, of the formula (VII) 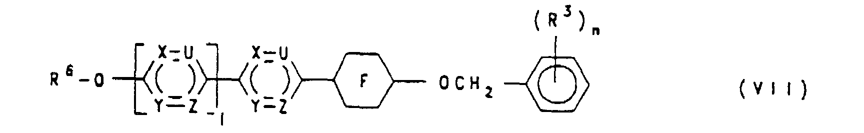 in which 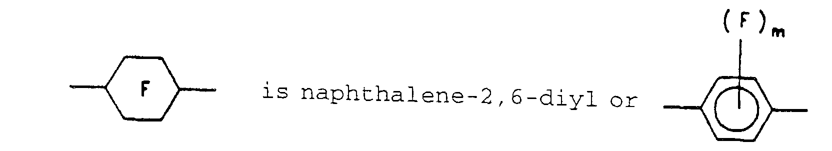
X, Y, U and Z are -CH=, -CF= and -N=, with the proviso that -CF= and -N= may each only be represented at most twice per six-membered ring and that -CF= and-N= must not simultaneously be represented twice in one six-membered ring, and that at least one nitrogen atom must be present in the ring bonded to R⁶-O-, and that X and/or Y must not be N in this ring;
R³ is CH₃ or OCH₃;
R⁶ is alkyl having 1 to 18 carbon atoms, in which, in addition, one or more non-adjacent -CH₂-groups may be replaced by -O-, -CH=CH- or -Si(CH₃)₂-,
l is 0 or 1; and
m and n are 0, 1, 2 or 3.

11. An intermediate, for the preparation of components for liquid-crystal mixtures, of the formula (VIII) 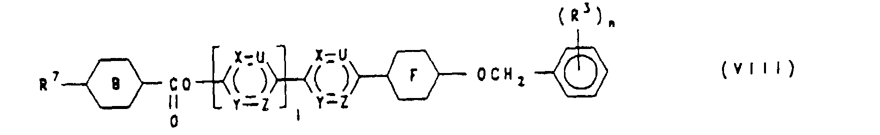 in which 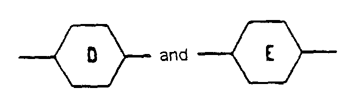 are naphthalene-2,6-diyl or 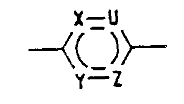 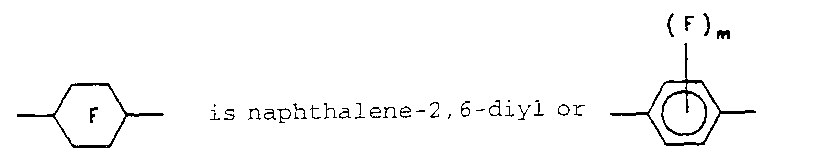 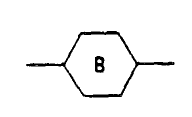 is 1,4-phenylene, unsubstituted or monosubstituted or disubstituted by F, or 1,4-cyclohexylene or 1,3-dioxane-2,5-diyl;
U, X, Y and Z are -CH=, -CF= and -N=, with the proviso that -CF= and -N= may each only be represented twice per six-membered ring, and -CF= and -N= must not simultaneously be represented twice in one six-membered ring;
R³ is CH₃ or OCH₃
R⁷ is alkyl having 1 to 18 carbon atoms, in which, in addition, one or more non-adjacent -CH₂-groups may be replaced by -O-, -CH=CH- or -Si(CH₃)₂;
l is 0 or 1; and
m and n are 0, 1, 2 or 3.

12. An intermediate, for the preparation of components for liquid-crystal mixtures, of the formula (XIII) 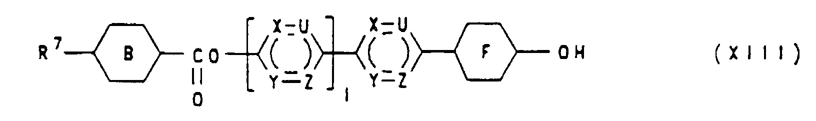 in which the symbols are as defined in the formula (VIII) in claim 11.

## Revendications (Revendications pour l'(les) Etat(s) contractant(s) suivant(s): DE, FR, GB)

1. Composés bifonctionnels de formule (I) 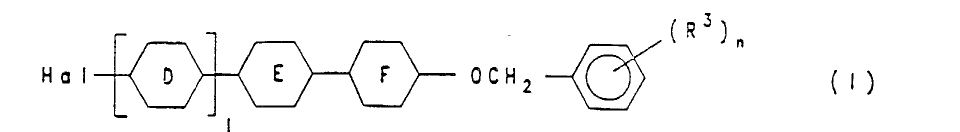 dans laquelle : 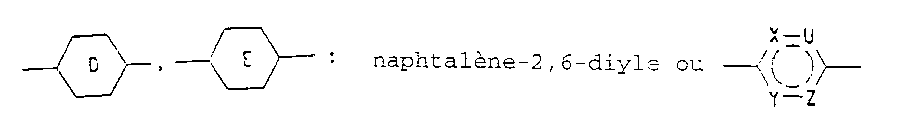 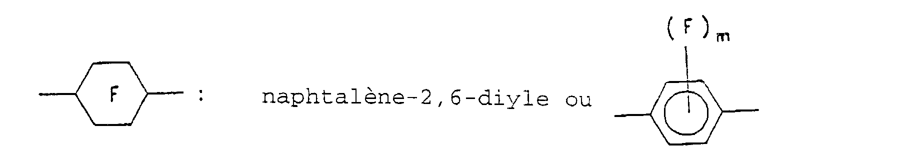
U, X, Y, Z : -CH=, -CF= et -N=, à la condition que -CF=, respectivement -N= ne puissent à chaque fois être présents que deux fois au maximum par cycle à 6 chaînons et que dans un cycle à six chaînons, -CF= et -N= ne puissent pas être présents deux fois simultanément ;
Hal : représente CI, Br ou I ; ou H, lorsque X ou/et Y représentent -CF=,
R³ : représente CH₃ ou OCH₃,
l : vaut 0 ou 1,
m, n : valent 0, 1, 2 ou 3,
le composé suivant étant exclu :

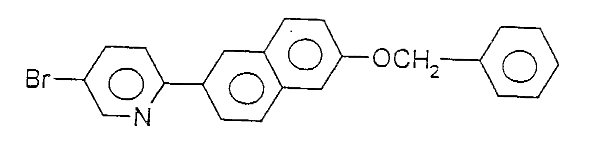

2. Composés bifonctionnels selon la revendication 1, caractérisés en ce que les symboles dans la formule générale I représentent
I = 0,
U, X, Y, Z : au moins une fois -N= et au maximum une fois -CF=, autrement -CH=,
m : 0, 1, 2 ou 3,
n : 0, 1 ou 2,
R³ : CH₃ ou OCH₃,
Hal : Br ou I.

3. Composés bifonctionnels selon la revendication 1 ou 2, caractérisés en ce que les symboles dans la formule générale I représentent
I = 0,
U, X, Y, Z : une ou deux fois -N=, autrement -CH=,
m : 0, 1, 2 ou 3,
n : 0,
Hal : Br ou I.

4. Procédé de préparation des composés bifonctionnels selon une ou plusieurs des revendications 1 à 3, caractérisés en ce que l'on couple un acide arylboronique de formule générale XXI, 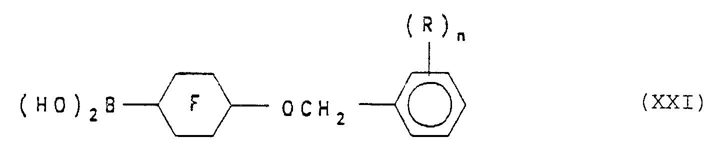 dans laquelle les substituants et les indices présentent les significations suivantes : 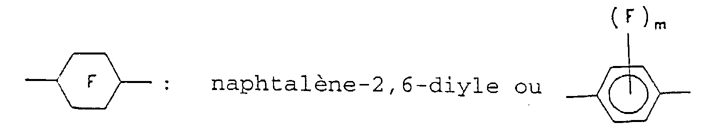
R : CH₃, OCH₃,
m : 0, 1, 2 ou 3,
n : 0, 1 ou 2,
avec un composé halogéné de formule générale XXII 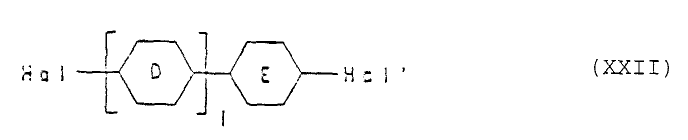 dans laquelle
Hal : CI, Br, I et
Hal' : Cl, Br, I et perfluoroalcane sulfonate,
sous catalyse par du palladium ou un composé de palladium, pour obtenir un composé de formule générale I.

5. Utilisation de composés bifonctionnels selon une ou plusieurs des revendications 1 à 3 comme produits intermédiaires pour la préparation de composants de mélanges de cristaux liquides.

6. Utilisation selon la revendication 5, caractérisée en ce que le mélange de cristaux liquides est ferroélectrique.

7. Utilisation selon la revendication 5, caractérisée en ce que le mélange de cristaux liquides est nématique.

8. Utilisation de composés bifonctionnels selon une ou plusieurs des revendications 1 à 3, comme composants de mélanges de cristaux liquides.

9. Produit intermédiaire de composants pour mélanges de cristaux liquides, de formule (VI) 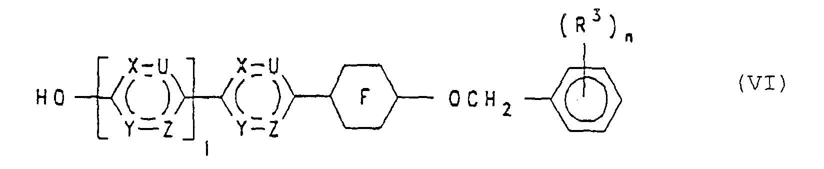 les symboles dans la formule I ayant les significations données dans la revendication 1 et pour le cycle porteur de la fonction OH, U et/ou Z représentent -N=, X et/ou Y ne doivent toutefois pas représenter -N=.

10. Produits intermédiaires pour la préparation de composants pour mélanges de cristaux liquides de formule (VII) 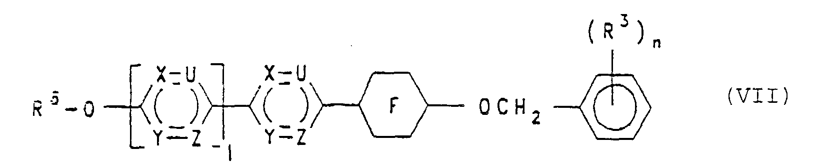 dans laquelle 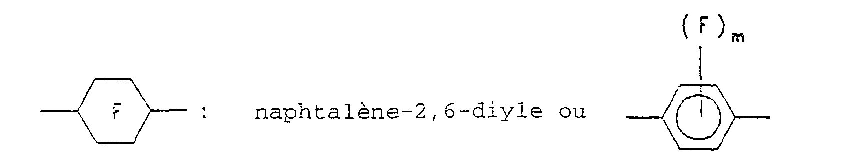
U, X, Y, Z : -CH=, -CF= et -N=, à la condition que -CF=, respectivement -N= ne puissent à chaque fois être présents que deux fois au maximum par cycle à 6 chaînons et que dans un cycle à six chaînons, -CF= et -N= ne puissent pas être présents deux fois simultanément et que dans R⁶-O lié au cycle au moins un atome d'azote doit être présent et que dans ce cycle X et/ou Y ne doivent pas être N ;
R³ : représente CH₃ ou OCH₃,
R⁶: représente alkyle avec 1 à 18 atomes de carbone, dans lequel un ou plusieurs groupes -CH₂- non adjacents pouvant être remplacés par -O-, -CH=CH- ou -Si(CH₃)₂ -,
l : vaut 0 ou 1,
m, n : valent 0, 1, 2 ou 3.

11. Produits intermédiaires, pour la préparation de composants pour mélanges de cristaux liquides, de formule (VIII) 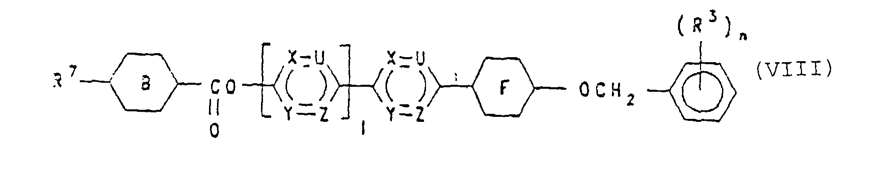 dans laquelle 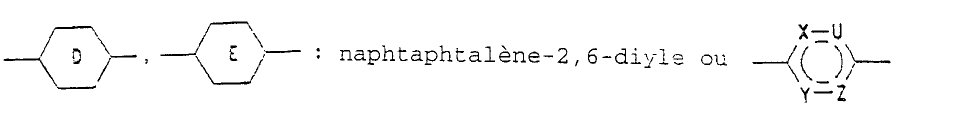 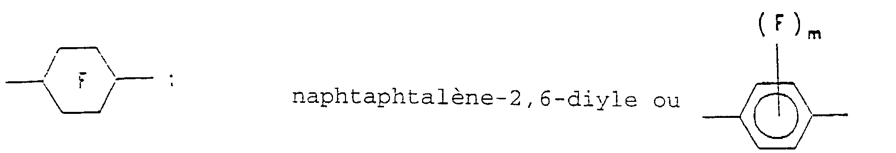 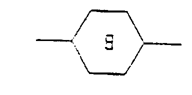 représente 1,4-phénylène, éventuellement 1 ou 2 fois substitué par f ou 1,4-cyclohexylène ou 1,3-dioxanne-2,5-diyle.
U, X, Y, Z : -CH=, -CF= et -N=, à la condition que -CF=, respectivement -N= ne puissent à chaque fois être présents que deux fois au maximum par cycle à 6 chaînons et que dans un cycle à 6 chaînons -CF= et -N= ne puissent pas être présent que deux fois simultanément ;
R³ : représente CH₃ ou OCH₃,
R⁷ : représente alkyle avec 1 à 18 atomes de carbone, dans lequel un ou plusieurs groupes -CH₂- non adjacents pouvant être remplacés par -O-, -CH=CH- ou -Si(CH₃)₂)-,
l : vaut 0 ou 1,
m, n : valent 0, 1, 2 ou 3.

12. Produits intermédiaires pour la préparation de composants pour mélanges de cristaux liquides, de formule (XIII), 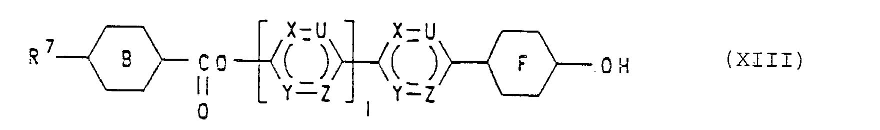 dans laquelle les symboles ont les significations données dans la formule (VIII) dans la revendication 11.

## Revendications (Revendications pour l'(les) Etat(s) contractant(s) suivant(s): CH, LI)

1. Composés bifonctionnels de formule (I) 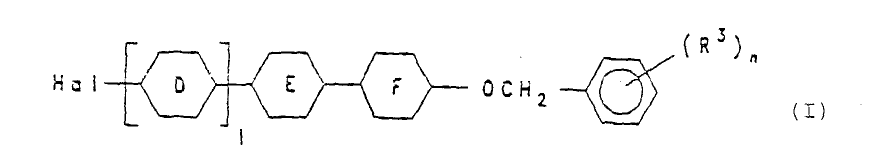 dans laquelle : 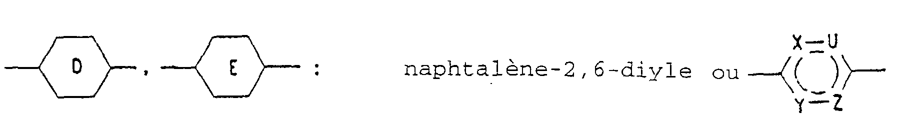 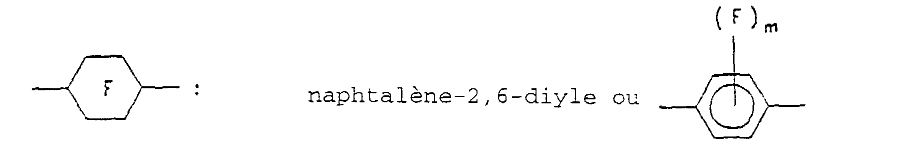
U, X, Y, Z : -CH=, -CF= et -N=, à la condition que -CF=, respectivement -N= ne puissent à chaque fois être présents que deux fois au maximum par cycle à 6 chaînons et que dans un cycle à six chaînons, -CF= et -N= ne puissent pas être présents deux fois simultanément ;
Hal : représente Cl, Br ou I ; ou H, lorsque X ou/et Y représentent -CF=,
R³ : représente CH₃ ou OCH₃,
l : vaut 0 ou 1,
m, n : valent 0, 1, 2 ou 3,

2. Composés bifonctionnels selon la revendication 1, caractérisés en ce que les symboles dans la formule générale I représentent
I = 0,
U, X, Y, Z : au moins une fois -N= et au maximum une fois -CF=, autrement -CH=,
m : 0, 1, 2 ou 3,
n : 0, 1 ou 2,
R³ : CH₃ ou OCH₃,
Hal : Br ou I.

3. Composés bifonctionnels selon la revendication 1 ou 2, caractérisés en ce que les symboles dans la formule générale I représentent
I = 0,
U, X, Y, Z : une ou deux fois -N=, autrement -CH=,
m : 0, 1, 2 ou 3,
n : 0,
Hal : Br ou I.

4. Procédé de préparation des composés bifonctionnels selon une ou plusieurs des revendications 1 à 3, caractérisé en ce que l'on couple un acide arylboronique de formule générale XXI, 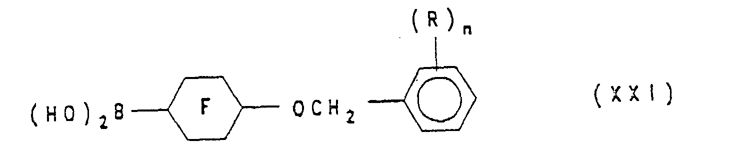 dans laquelle les substituants et les indices présentent les significations suivantes : 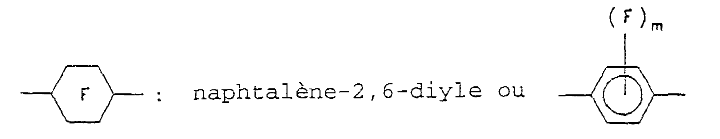
R : CH₃, OCH₃,
m : 0, 1, 2 ou 3,
n : 0, 1 ou 2,
avec un composé halogéné de formule générale XXII 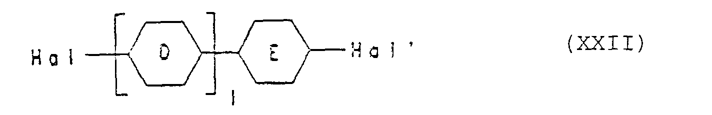 dans laquelle
Hal : Cl, Br, I et
Hal' : Cl, Br, I et perfluoroalcane sulfonate,
catalysée par le palladium ou un composé de palladium, pour obtenir un composé de formule générale I.

5. Utilisation des composés bifonctionnels selon une ou plusieurs des revendications 1 à 3 comme produits intermédiaires pour la préparation de composants de mélanges de cristaux liquides.

6. Utilisation selon la revendication 5, caractérisée en ce que le mélange de cristaux liquides est ferroélectrique.

7. Utilisation selon la revendication 5, caractérisée en ce que le mélange de cristaux liquides est nématique.

8. Utilisation des composés bifonctionnels selon une ou plusieurs des revendications 1 à 3, comme composants de mélanges de cristaux liquides.

9. Produit intermédiaire pour la préparation de composants pour mélanges de cristaux liquides, de formule (VI) 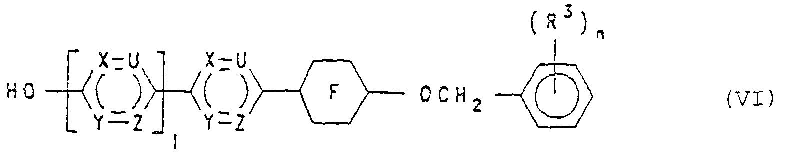 les symboles dans la formule I ayant les significations données dans la revendication 1 et pour le cycle porteur de la fonction OH, U et/ou Z représentent -N=, X et/ou Y ne doivent toutefois pas représenter -N=.

10. Produits intermédiaires pour la préparation de composants pour mélanges de cristaux liquides de formule (VII) 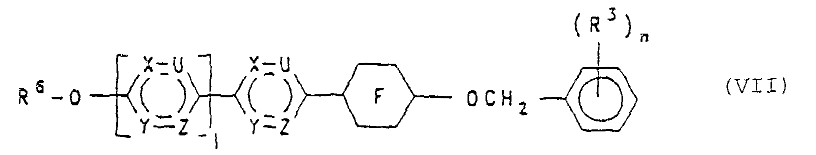 dans laquelle 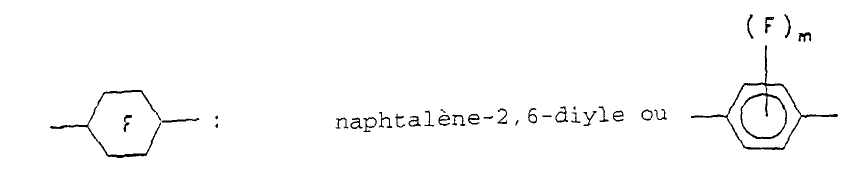
U, X, Y, Z : -CH=, -CF= et -N=, à la condition que -CF=, respectivement -N= ne puissent à chaque fois être présents que deux fois au maximum par cycle à 6 chaînons et que dans un cycle à six chaînons, -CF= et -N= ne puissent pas être présents deux fois simultanément et que dans R⁶-O lié au cycle au moins un atome d'azote doit être présent et que dans ce cycle X et/ou Y ne doivent pas être N ;
R³ : représente CH₃ ou OCH₃,
R⁶: représente alkyle avec 1 à 18 atomes de carbone, dans lequel un ou plusieurs groupes -CH₂- non adjacents pouvant être remplacés par -O-, -CH=CH- ou -Si(CH₃)₂ -,
l : vaut 0 ou 1,
m, n : valent 0, 1, 2 ou 3.

11. Produits intermédiaires, pour la préparation de composants pour mélanges de cristaux liquides, de formule (VIII) 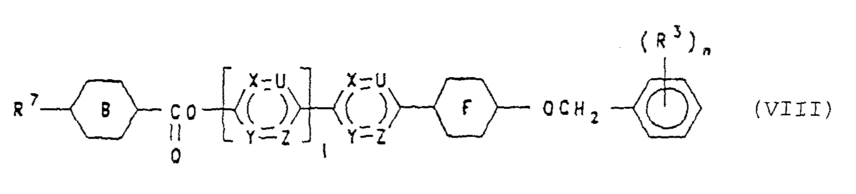 dans laquelle 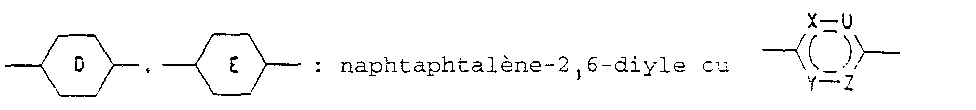 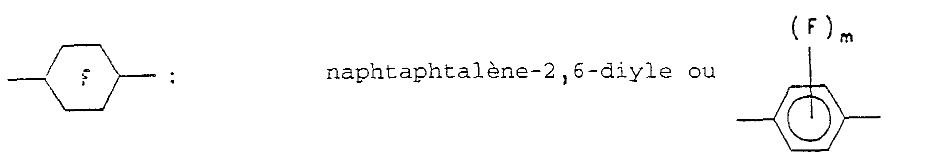 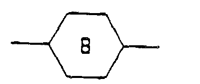 représente 1,4-phénylène, éventuellement 1 ou 2 fois substitué par F, ou 1,4-cyclohexylène ou 1,3-dioxanne-2,5-diyle.
U, X, Y, Z : -CH=, -CF= et -N=, à la condition que -CF=, respectivement -N= ne puissent à chaque fois être présents que deux fois au maximum par cycle à 6 chaînons et que dans un cycle à 6 chaînons -CF= et -N= ne puissent pas être présentx deux fois simultanément ;
R³ : représente CH₃ ou OCH₃,
R⁷ : représente alkyle avec 1 à 18 atomes de carbone, dans lequel un ou plusieurs groupes -CH₂- non adjacents pouvant être remplacés par -O-, -CH=CH- ou -Si(CH₃)₂)-,
l : vaut 0 ou 1,
m, n : valent 0, 1, 2 ou 3.

12. Produits intermédiaires pour la préparation de composants pour mélanges de cristaux liquides, de formule (XIII), 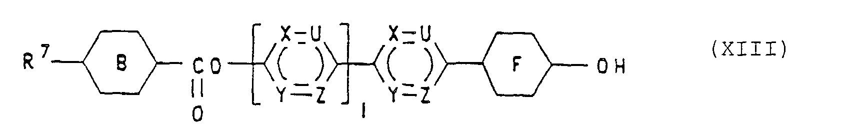 dans laquelle les symboles ont les significations données dans la formule (VIII) dans la revendication 11.
